# EUROPEAN PATENT APPLICATION

(11) **EP 4 062 909 A1**
(43) Date of publication of application: **28.09.2022**
(21) Application number: 21165307.6
(22) Date of filing: 26.03.2021
(51) Int. Cl.: A61K 31/341, A61K 31/433, A61K 45/06, G01N 33/50, A61P 35/00

(54) **CARBONIC ANHYDRASE 1 (CA1) INHIBITORS FOR THE TREATMENT OR PREVENTION OF MYELOPROLIFERATIVE DISORDERS AND OTHER HEMATOPOIETIC MALIGNANCIES, AND AS BIOMARKER OF MYELOPROLIFERATIVE DISORDERS AND OTHER HEMATOPOIETIC MALIGNANCIES**

(71) Applicant: Commissariat à l'Energie Atomique et aux Energies Alternatives, 75015 Paris (FR); INSTITUT NATIONAL DE LA SANTE ET DE LA RECHERCHE MEDICALE - INSERM, 75013 Paris (FR); Université de Paris, 75006 Paris (FR); Université Paris-Saclay, 91190 Saint-Aubin (FR)
(72) Inventor: ROMEO, Paul-Henri, 92290 Fontenay-aux Roses (FR); LEWANDOWSKI, Daniel, 92290 Fontenay-aux Roses (FR); BARROCA, Vilma, 92290 Fontenay-aux Roses (FR); MURAKAMI, Shohei, 92290 Fontenay-aux Roses (FR)
(74) Representative: Nony

(57) **Abstract**

The invention relates to the treatment or prevention of myeloproliferative disorders and other hematopoietic malignancies.

Herein is described a carbonic Anhydrase 1 (CA1) inhibitor; for use in a method for treating or preventing a myeloproliferative disorder, an acute myeloid leukemia (AML) and/or a primary or secondary myelofibrosis.

Herein is further described the use of carbonic Anhydrase 1 as a biomarker for myeloproliferative disorders and other hematopoietic malignancies; and/or as a biomarker of the efficacy of compounds for treating or preventing said conditions.

## Description

### TECHNICAL FIELD

The invention relates to the treatment or prevention of myeloproliferative disorders and other hematopoietic malignancies.

Herein is described a Carbonic Anhydrase 1 (CA1) inhibitor; for use in a method for treating or preventing a myeloproliferative disorder, an acute myeloid leukemia (AML) and/or a primary or secondary myelofibrosis.

Herein is further described the use of carbonic Anhydrase 1 as a biomarker for myeloproliferative disorders and other hematopoietic malignancies; and/or as a biomarker of the efficacy of compounds for treating or preventing said conditions.

### BACKGROUND OF THE INVENTION

Hematopoietic malignancies have been modeled using transgenic and knock-in models or bone marrow transplantation of mutated hematopoietic cells, as shown in Akada, H. et al. (Conditional expression of heterozygous or homozygous Jak2V617F from its endogenous promoter induces a polycythemia vera-like disease. Blood 115, 3589-3597 (2010)) or Wernig, G. et al. (Expression of Jak2V617F causes a polycythemia vera-like disease with associated myelofibrosis in a murine bone marrow transplant model. Blood 107, 4274-4281 (2006)) for JAK2^{V617F} mutations.

However, these models result in the expression of mutated gene(s), such as JAK2^{V617F} mutations, in targeted hematopoietic cells or require the use of total body irradiation (TBI) or immunodeficient mice. Thus, how a hematopoietic malignancy can progressively invade hematopoietic organs from a defined unique anatomical site cannot be studied in immune-competent mice.

In particular, JAK2^{V617F} mutation is found in more than 90% of Polycythemia Vera (PV) patients, as described in Ugo et al. (A unique clonal JAK2 mutation leading to constitutive signalling causes Polycythemia Vera. Medecine/Sciences 21, 669-670 (2005)) and in more than 50% of patients with Essential Thrombocytemia (ET) and Primary MyeloFibrosis (PMF); as evidenced in Baxter et al. (Acquired mutation of the tyrosine kinase JAK2 in human myeloproliferative disorders. Lancet 365, 1054-1061 (2005)) and Levine et al. (Activating mutation in the tyrosine kinase JAK2 in polycythemia vera, essential thrombocythemia, and myeloid metaplasia with myelofibrosis. Cancer Cell 7, 387-397 (2005)). Mouse models of these myeloproliferative neoplasms (MPN) have been useful to characterize JAK2 specific inhibitors for MPN treatment that were then used to treat patients; as described in Wernig et al. (Efficacy of TG101348, a Selective JAK2 Inhibitor, in Treatment of a Murine Model of JAK2V617F-Induced Polycythemia Vera. Cancer Cell 13, 311-320 (2008)). Unfortunately, none of these treatments targets the malignant clone and after initial beneficial response, many patients lose responsiveness to these inhibitors and develop adverse effects.

For instance, the JAK1/JAK2 inhibitor ruxolitinib is one of the most used treatment of Polycythemia Vera (PV) but it has no major effect on JAK2^{V617F} cell burden and many patients develop cytopenia due to its non-selective effect on JAK2^{V617F}.

US5035878 teaches compounds for treating myeloproliferative disorders.

US10639382 teaches compounds for the treatment of chronic myeloid leukemias (CML) and acute myeloid leukemias (AML). It also discloses an *in vitro* screening for a set of leukemic categories including CML, chronic lymphocytic leukemias (CLL), AML and acute lymphocytic leukemia (ALL). However, the screening provides distinct outcomes between myeloid and lymphocytic leukemias for a set of prospective active agents.

Thus, there is still a need to explore new therapeutic targets for hematopoietic malignancies, and more particularly myeloproliferative disorders such as PV.

There is also a need for biomarkers for targeting such hematopoietic malignancies, and/or for screening new therapeutic compounds.

### SUMMARY OF THE INVENTION

According to a **first main embodiment,** the invention relates to a carbonic Anhydrase 1 (CA1) inhibitor; for use in a method for treating or preventing a myeloproliferative disorder, an acute myeloid leukemia (AML) and/or a primary or secondary myelofibrosis.

In particular, the invention relates to the carbonic anhydrase 1 (CA1) inhibitor; for use in a method as previously described; wherein the CA1 inhibitor is a sulphonamide or a pharmaceutically acceptable salt thereof.

In particular, the invention relates to the carbonic anhydrase 1 (CA1) inhibitor; for use in a method as previously described; wherein the CA1 inhibitor is selected from Furosemide, Acetazolamide, and pharmaceutically acceptable salts thereof.

In particular, the invention relates to the carbonic anhydrase 1 (CA1) inhibitor; for use in a method as previously described; wherein the CA1 inhibitor is selected from Furosemide and pharmaceutically acceptable salts thereof.

In particular, the invention relates to the carbonic anhydrase 1 (CA1) inhibitor; for use in a method as previously described; wherein the CA1 inhibitor is selected from Acetazolamide and pharmaceutically acceptable salts thereof.

According to particular embodiments, the invention relates to the carbonic anhydrase 1 (CA1) inhibitor; for use in a method as previously described; wherein the hematopoietic disorder is selected from: a chronic myeloid leukemia (CML), a chronic neutrophilic leukemia (CNL), polycythemia vera (PV), a primary myelofibrosis (PMF), an essential thrombocythemia (ET), a chronic eosinophilic leukemia.

According to particular embodiments, the invention relates to the carbonic anhydrase 1 (CA1) inhibitor; for use in a method as previously described; wherein it is for treating or preventing a polycythemia vera (PV), an acute myeloid leukemia (AML), a primary myelofibrosis (PMF) and/or a secondary myelofibrosis.

According to particular embodiments, the invention relates to the carbonic anhydrase 1 (CA1) inhibitor; for use in a method as previously described; wherein it is for treating or preventing a primary myelofibrosis (PMF) or a secondary myelofibrosis.

According to a **second main embodiment,** the invention relates to a pharmaceutical composition comprising a Carbonic Anhydrase 1 (CA1) inhibitor; for use in a method for treating or preventing a myeloproliferative disorder, an acute myeloid leukemia (AML) and/or a primary or secondary myelofibrosis.

According to a **third main embodiment,** the invention relates to an *in vitro* or *ex vivo* use of a level of expression or activity of Carbonic Anhydrase 1 (CA1), as a biomarker of a myeloproliferative disorder, an acute myeloid leukemia (AML) and/or a primary or secondary myelofibrosis.

According to a **fourth main embodiment,** the invention relates to an *in vitro* or *ex vivo* method for the diagnosis or follow-up of a myeloproliferative disorder, an acute myeloid leukemia (AML) and/or a primary or secondary myelofibrosis in an individual, comprising the steps of:
a) determining a level of expression or activity of Carbonic Anhydrase 1 (CA1) in a biological sample from the individual;
b) comparing the level of expression or activity of CA1 to a reference value, wherein an increase of the level of expression or activity of Carbonic Anhydrase 1 (CA1) is indicative of the occurrence of said myeloproliferative disorder, acute myeloid leukemia (AML) and/or primary or secondary myelofibrosis in the individual.

According to a **fifth main embodiment,** the invention relates to an *in vitro* or *ex vivo* method for screening an active agent for the treatment or prevention of a myeloproliferative disorder, an acute myeloid leukemia (AML) and/or a primary or secondary myelofibrosis, comprising the steps of:
a) determining a level of expression or activity of Carbonic Anhydrase 1 (CA1) in a biological sample;
b) comparing the level of expression or activity of CA1 to a reference value, wherein a decrease of the level of expression or activity of Carbonic Anhydrase 1 (CA1) in the biological sample by the active agent is indicative of its efficacy for the treatment or prevention of said myeloproliferative disorder, acute myeloid leukemia (AML) and/or primary or secondary myelofibrosis.

According to particular embodiments of the **fourth** and **fifth main embodiment,** the biological sample is a blood sample that comprises CD34-positive cells.

According to particular embodiments of the **fourth** and **fifth main embodiment,** the active agent is selected from sulphonamides and derivatives thereof; in particular Furosemide, Acetazolamide, and pharmaceutically acceptable salts thereof.

### BRIEF DESCRIPTION OF THE FIGURES

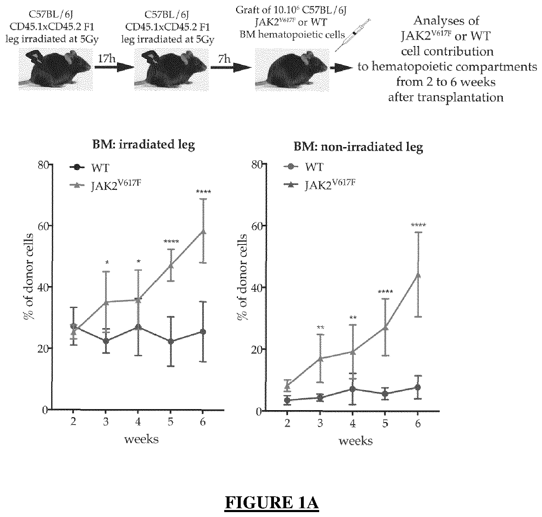

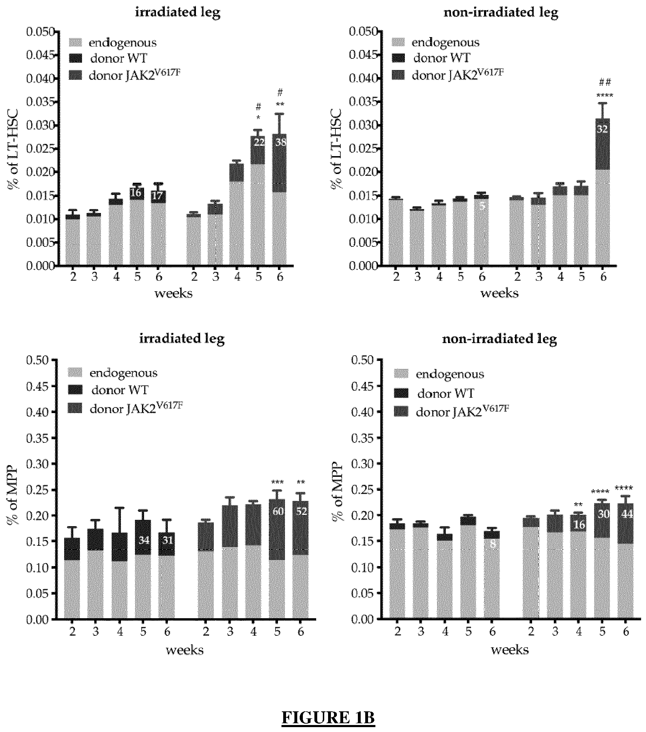

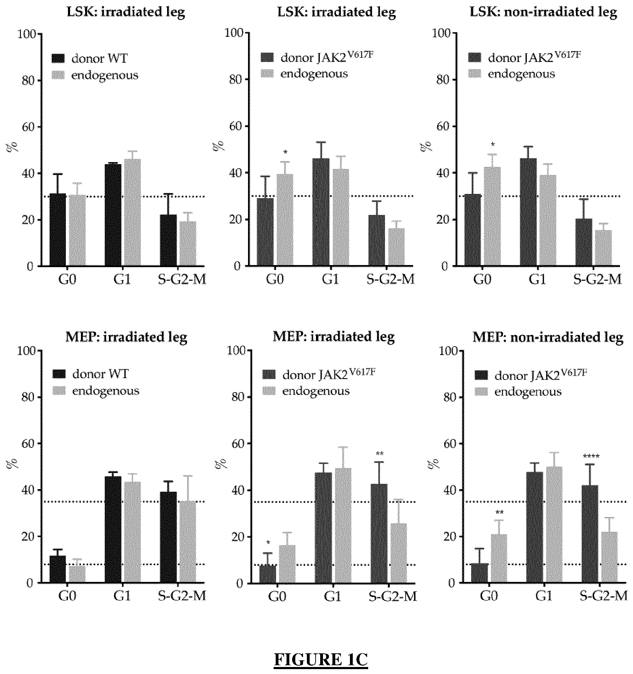

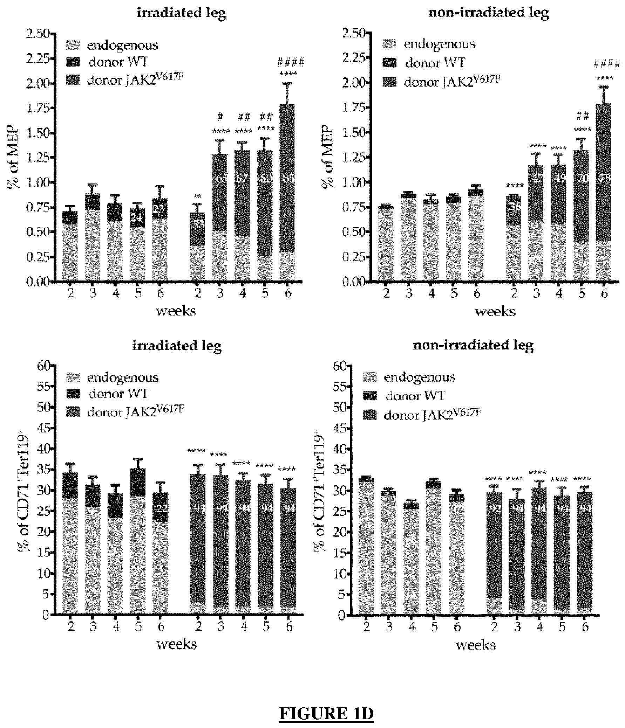

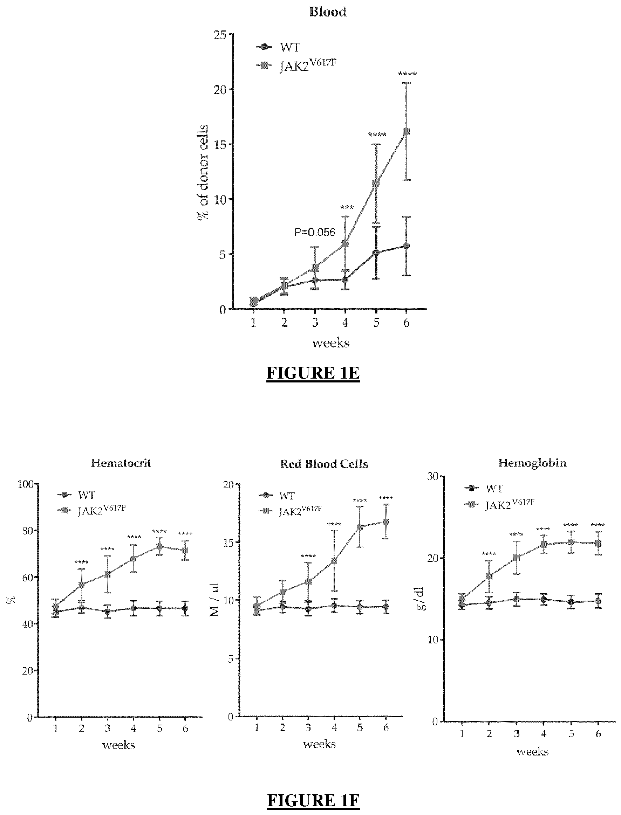

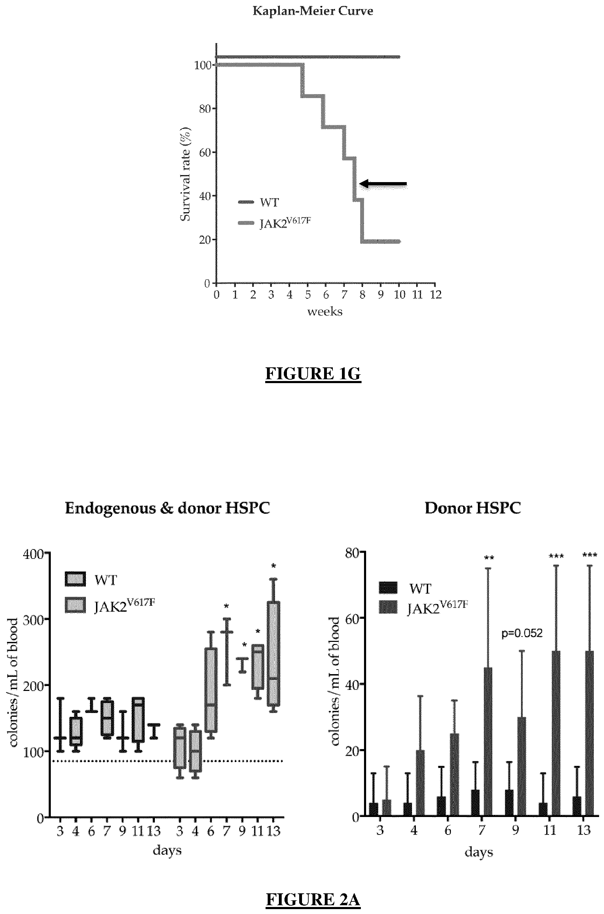

**Fig. 1****: Follow up of Polycythemia Vera development in mice.** Experimental design (**1a**) and kinetics of grafted WT or JAK2^{V617F} bone marrow (BM) cells contribution to total BM hematopoietic cells in the irradiated and non-irradiated legs, with contribution of grafted WT cells over endogenous cells at 2, 3, 4, 5 and 6 weeks on the left of each panel (**1b**) and contribution of grafted JAK2^{V617F} cells over endogenous cells at 2, 3, 4, 5 and 6 weeks on the right of each panel (**1b**), (**1c**), Kinetics of WT or JAK2^{V617F} cells contribution to the long term hematopoietic stem cell (LT-HSC), with legend as in fig. 1b **(up)** and the multipotent progenitor (MPP) **(bottom)** compartments, **1c**, Cell cycle analysis of WT (left) and JAK2^{V617F} (middle and right) Lin⁻cKit⁺Sca⁺ (LSK) **(up)** and megakaryocyte-erythroid progenitors (MEP) **(bottom)** and of their endogenous counterparts in the irradiated and non-irradiated legs 3 weeks after transplantation, **Id,** Kinetics of WT or JAK2^{V617F} cells contribution to MEP **(up)** and to CD71⁺Ter119⁺ mature erythroid **(bottom)** compartments in the irradiated and non-irradiated legs with legend as in fig. 1b, After transplantation of JAK2^{V617F} or WT BM cells in a single irradiated leg, kinetics of blood cells (**1e**), of blood parameters **(If)** and Kaplan-Mier survival curve of mice (**1g**), with a decrease of the survival rate for JAK2^{V617F} marked by an arrow.

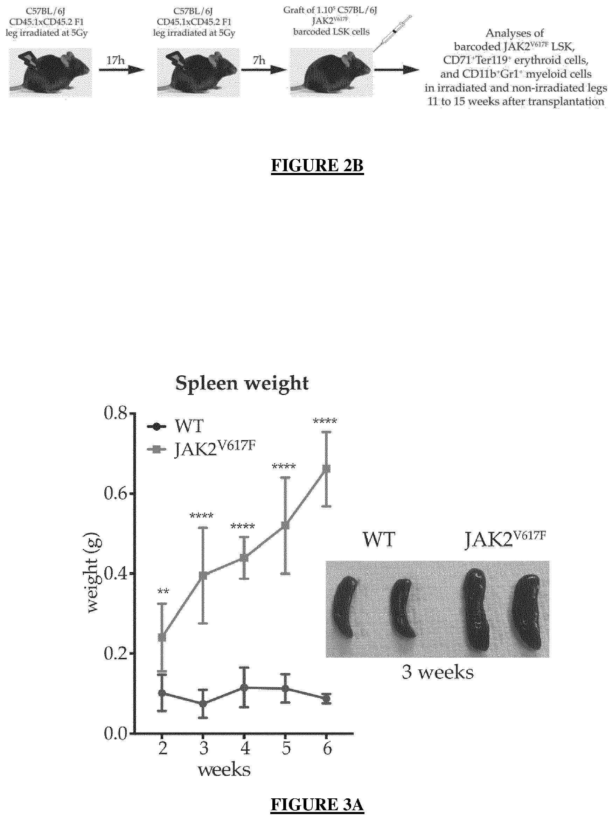

**Fig. 2****: JAK2^{V617F} LSK from irradiated leg colonize the non-irradiated leg. 2a,** Using methylcellulose assay, numbers of total and WT **(left column for each data set)** or JAK2^{V617F} **(right)** blood circulating Hematopoietic Stem and Progenitor Cells (HSPC) per ml of blood in mice grafted with JAK2^{V617F} or WT BM cells in a single irradiated leg, **2b,** Experimental design for establishment of the frequency of bare-coded JAK2^{V617F} LSK clones in the non-irradiated and irradiated legs.

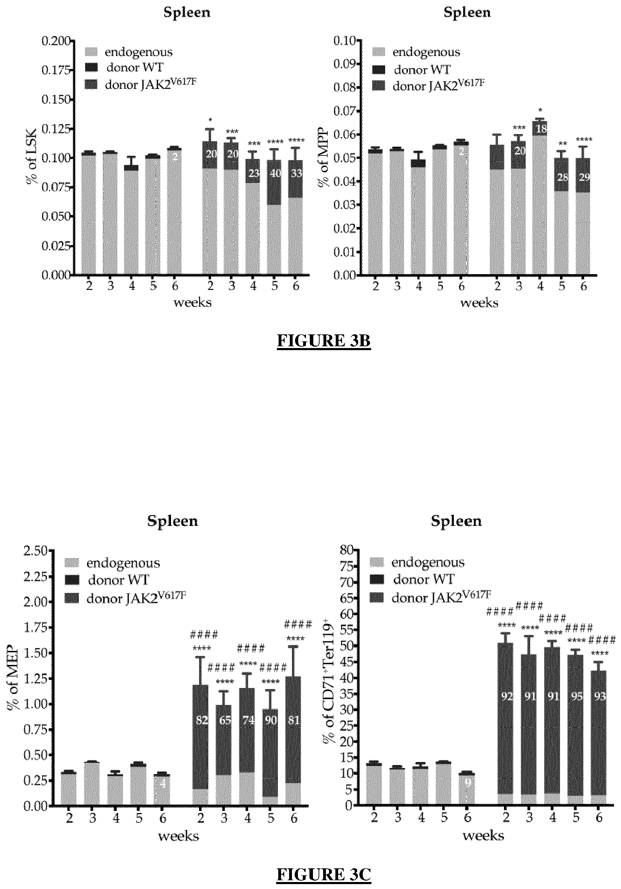

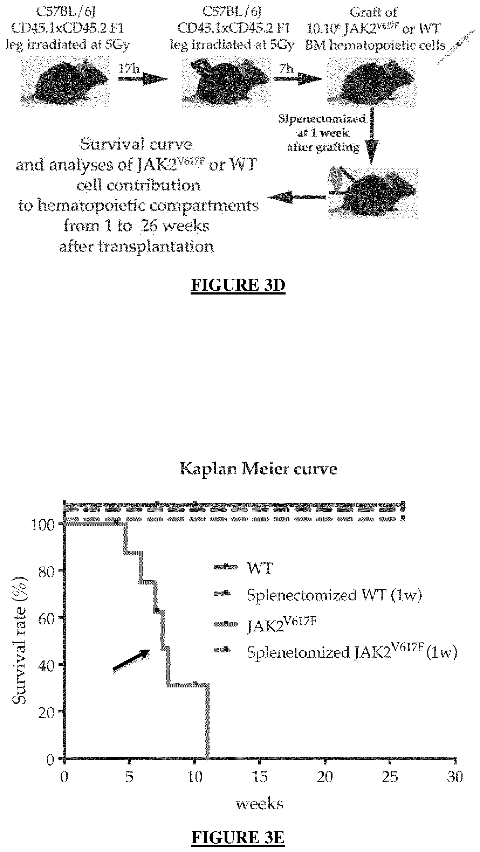

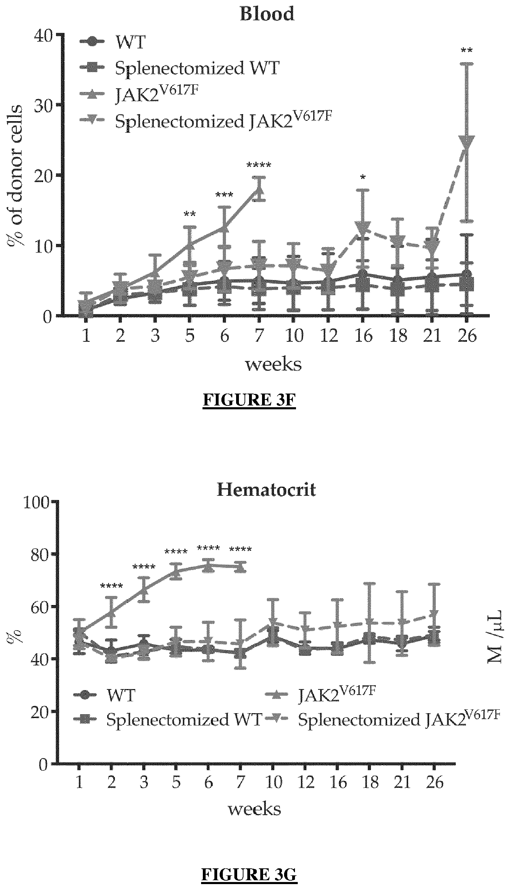

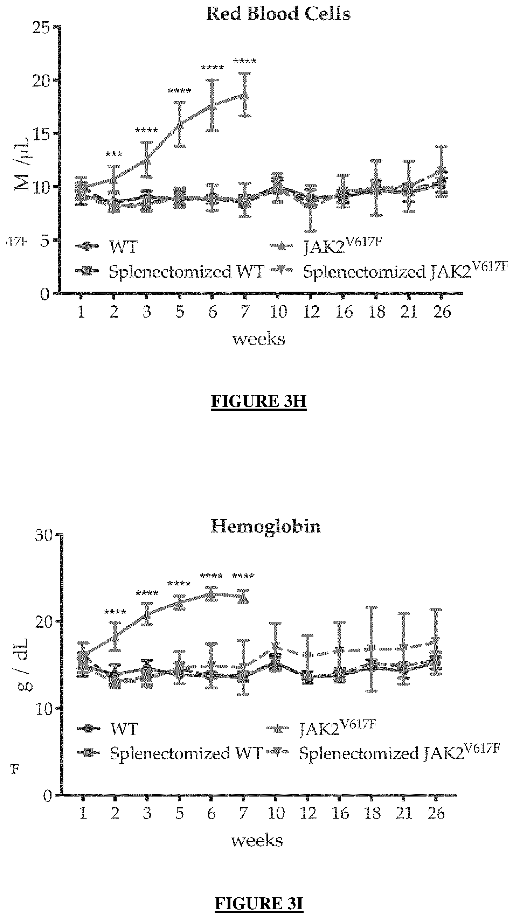

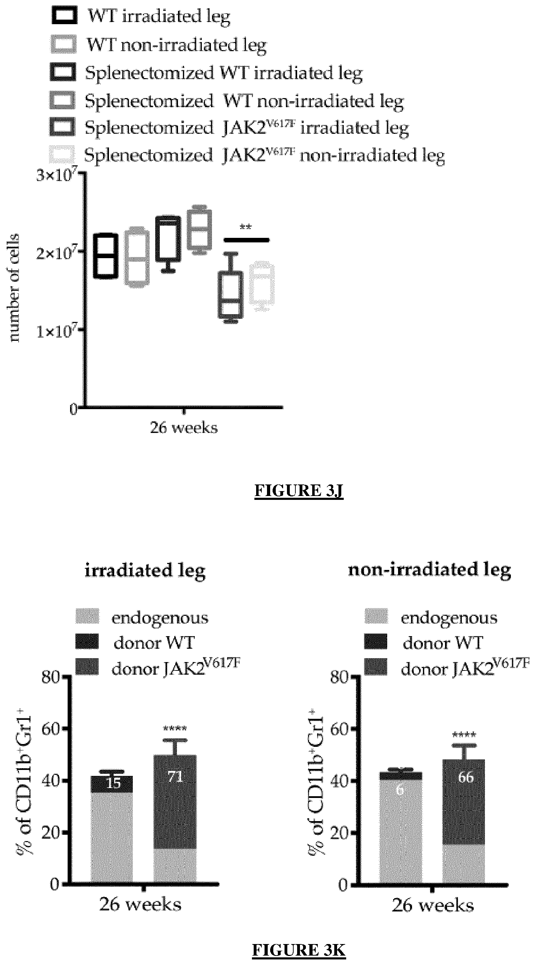

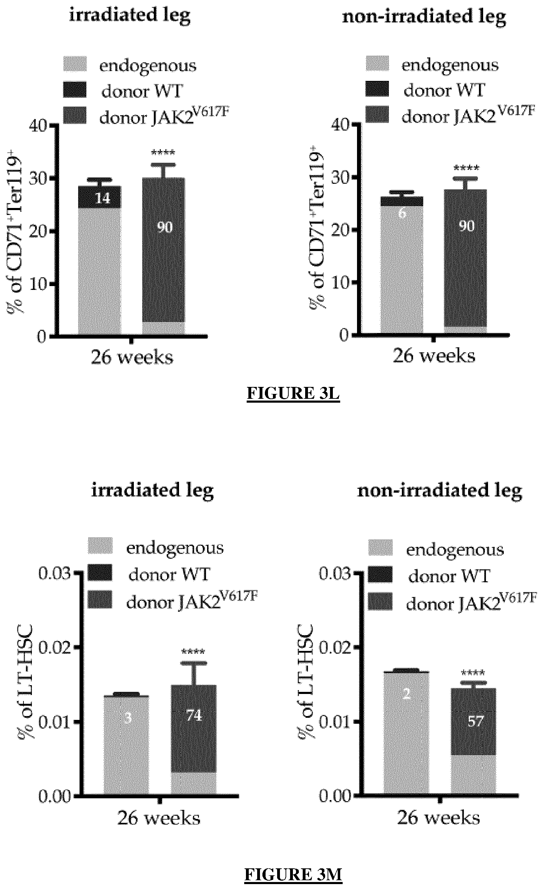

**Fig. 3****: Spleen is necessary for Polycythemia Vera development and acts as JAK2^{V617F} erythroid compartment amplifier. 3a,** Kinetics of spleens weight **(left)** and pictures of spleens 3 weeks after transplantation of WT and JAK2V^{617F} BM cells **(right),** 3b, Kinetics of WT or JAK2V^{617F} cells contribution to the LSK and multipotent progenitor (MPP) cell compartments with legend as in fig. 1b, and **3c****,** to the megakaryocyte-erythroid progenitor (MEP) and the CD71⁺Ter119⁺ erythroid compartments in the spleen with legend as in fig. 1b (contribution of WT cells over endogenous cells at 2, 3, 4, 5 and 6 weeks on the left of each panel), **3d** & **e,** Kaplan-Meier survival curve with a decrease of the survival rate for JAK2^{V617F} marked by an arrow, kinetics of blood cells, **3f to 3j,** total BM hematopoietic cells number and blood parameters (hematocrit, red blood cells and hemoglobin), **3j,** JAK2^{V617F}, WT and endogenous cells contributions, with splenectomized JAK2^{V617F} in irradiated and non-irradiated leg marked by ** as statistically significant, **3k**, the CD11b⁺Gr1⁺ myeloid, **3l,** the CD71⁺Ter119⁺ erythroid compartments and **3m,** the LT-HSC compartment 26 weeks after splenectomy.

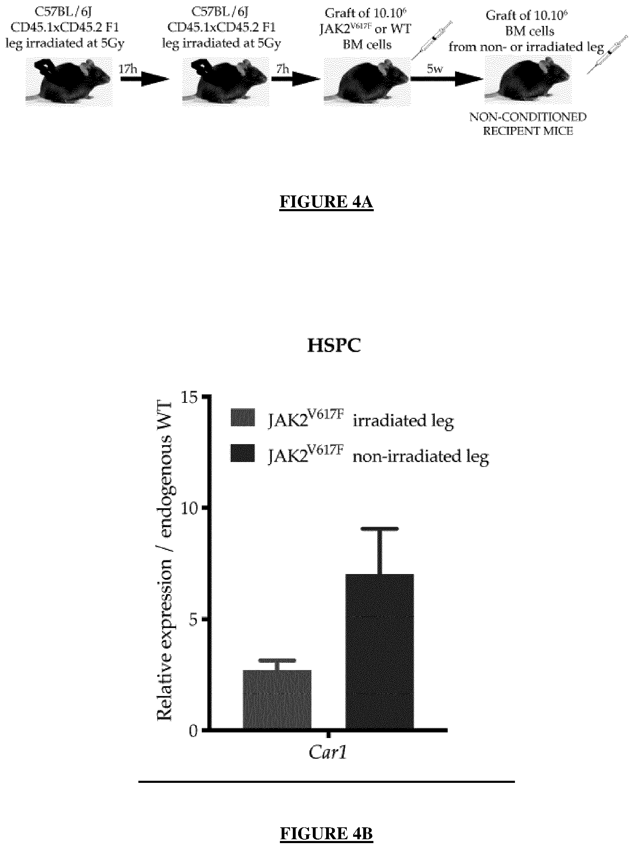

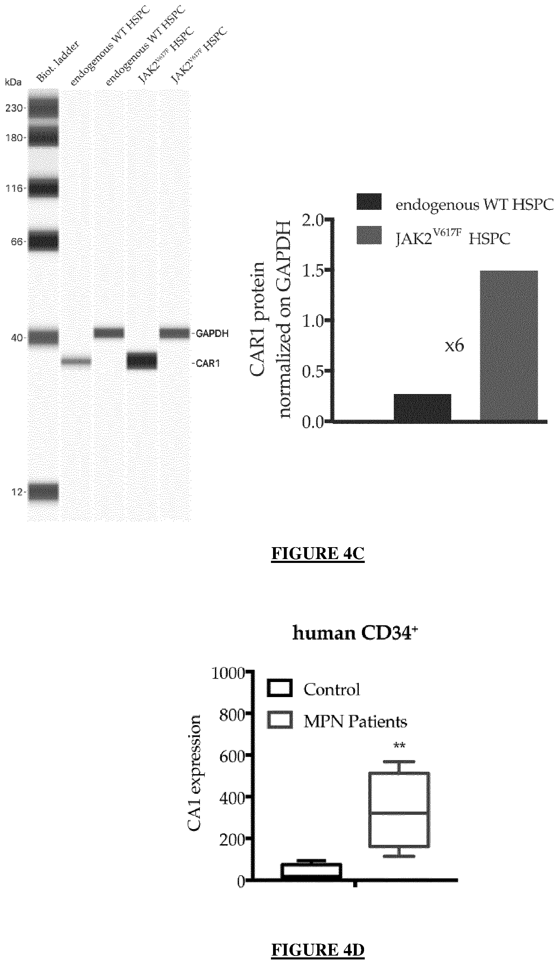

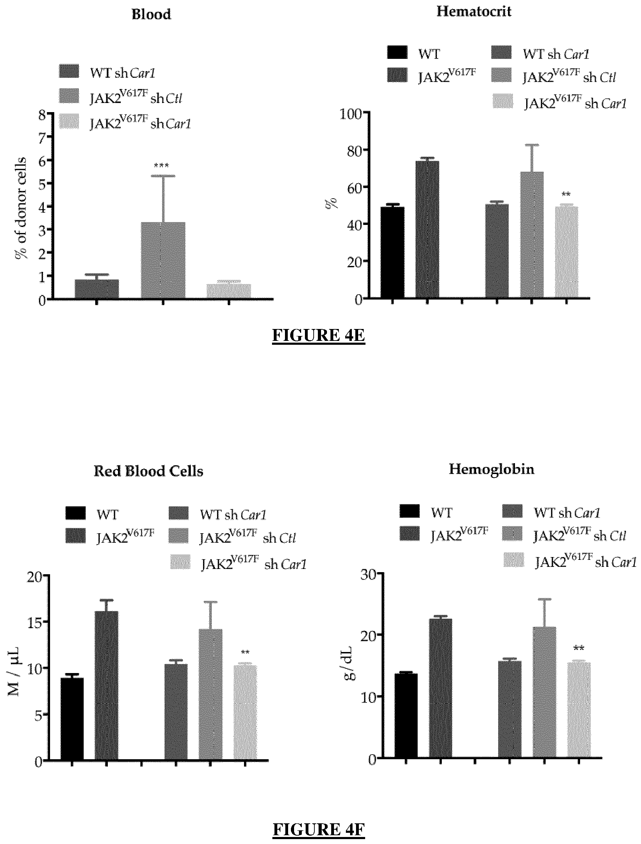

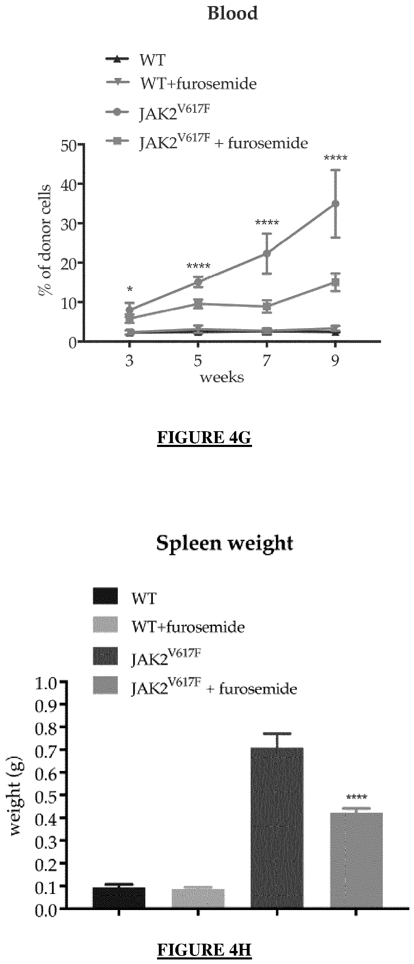

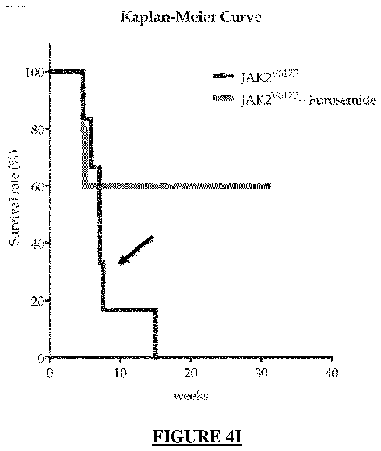

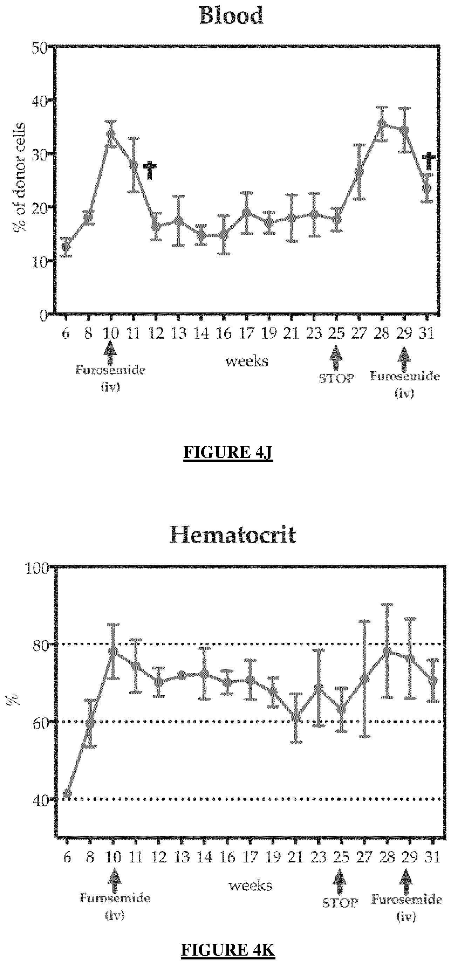

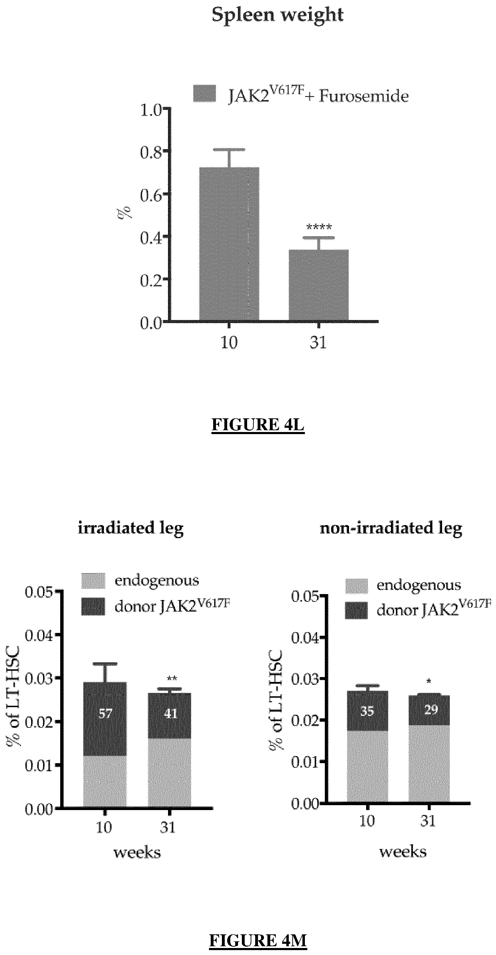

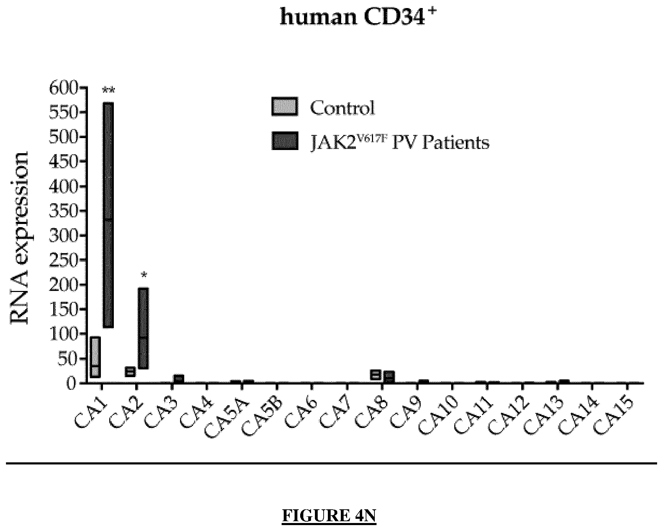

**Fig. 4****: Genetic and pharmacologic inhibition of carbonic anhydrase 1 suppresses Polycythemia Vera progression.** Unless specified otherwise, the legend lists from up to bottom the corresponding entries which appear from left to right on the graph. **4a,** Experimental design for determination of kinetics of blood chimerisms in immuno-competent mice transplanted with BM from either the irradiated leg or the non-irradiated leg from mice previously grafted in a single irradiated leg with JAK2^{V617F} or WT BM cells, and numbers of dead mice after these secondary transplantations, **4b,** relative mRNA level of carbonic anhydrase 1 in HSPC from irradiated (left column) and non-irradiated (right column) legs after 6 weeks after transplantation of JAK2^{V617F} BM cells, **(4c)** CAR1 protein expression in HSPC of the non-irradiated leg 6 weeks after transplantation of JAK2^{V617F} BM cells **(left)** normalized on GAPDH protein level **(right), (4d),** mRNA level of carbonic anhydrase 1 in human CD34⁺ cells from control and JAK2^{V617F} MPN patients, **(4e),** blood chimerism and blood parameters (blood and hematocrit) of a single leg irradiated mice grafted with JAK2^{V617F} or WT LSK cells transduced with *Carl* or *Ctl* shRNA, or **(4f),** corresponding red blood cells and hemoglobin. **(4g)** Kinetics of blood cells of mice grafted with JAK2^{V617F} or WT BM cells and treated or not with furosemide 2 weeks after transplantation. Effects of furosemide treatment starting 2 weeks after transplantation of JAK2^{V617F} or WT BM cells on **(h)** spleen weights 8 weeks later, and (i) survival rate curve with a decrease of the survival rate for JAK2^{V617F} without Furosemide marked by an arrow. Effects of furosemide treatments starting 10 weeks after transplantation of JAK2^{V617}F BM cells on chimerism in blood cells **(4j)** and hematocrit **(4k)** crosses indicated death of non-treated mice, **(4l),** spleen weight and **(4m),** JAK2^{V617F} cells contribution to the LT-HSC compartments of non-irradiated and irradiated legs before furosemide treatment (10 weeks) and at the end of furosemide treatment (31 weeks).

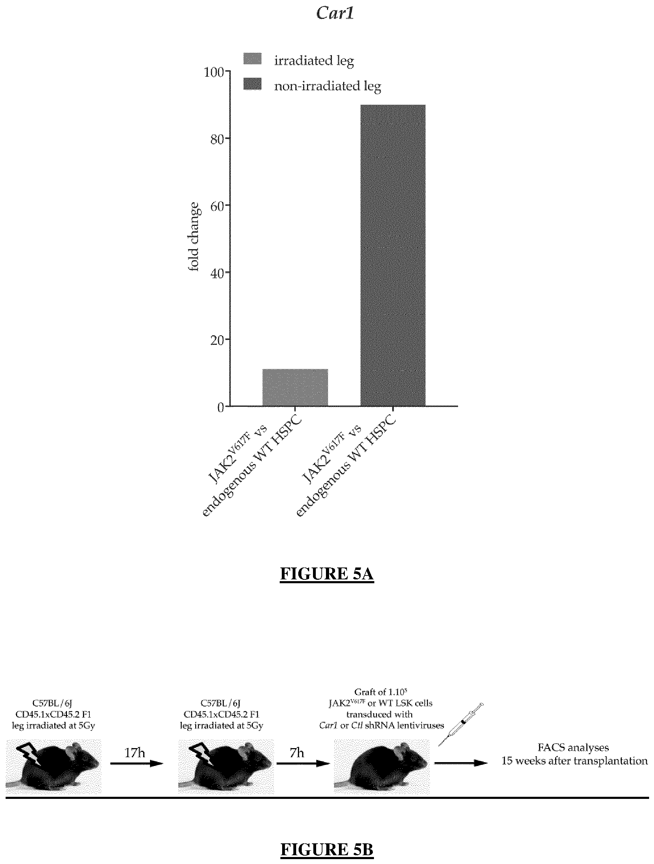

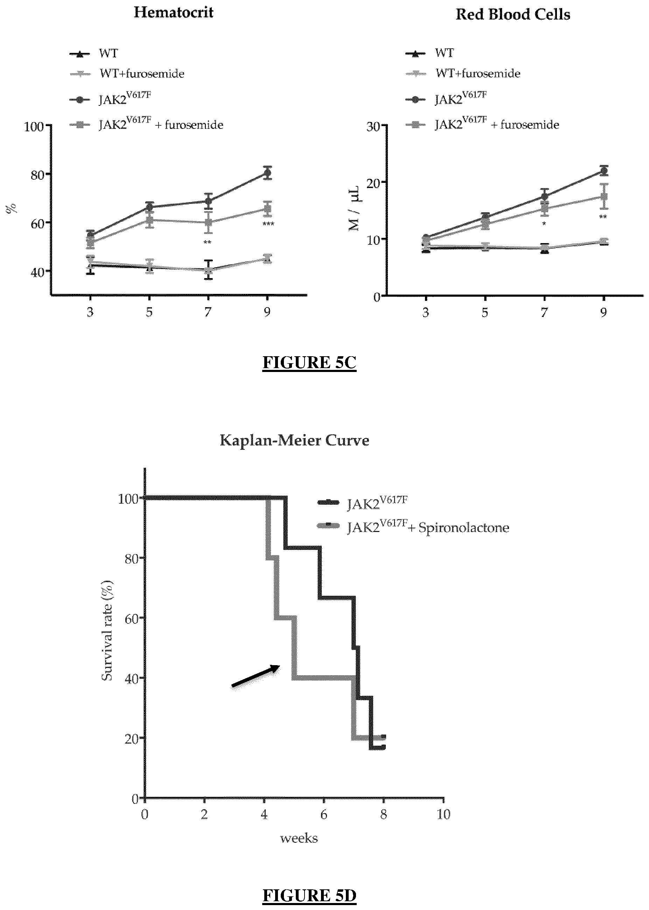

**Fig. 5****. Inhibition of carbonic anhydrase 1 suppresses Polycythemia Vera progression. 5a,** Fold change of carbonic anhydrase 1 mRNA levels in JAK2^{V617F} *versus* endogenous WT HSPC in irradiated (left column) and non-irradiated (right column) legs from the transcriptomic analysis, **(5b)** experimental design of a single leg irradiated mice transplanted with JAK2^{V617F} or WT LSK cells transduced with *Car1* or *Ctl* shRNA lentiviruses that can also express the Sherry gene under H1 promoter, **(5c)** Hematocrit and red blood cells count from a single leg irradiated mice grafted with JAK2^{V617F} or WT BM cells and treated or not with furosemide 2 weeks after transplantation, **(5d),** Survival curve of a single leg irradiated mice grafted with JAK2^{V617F} BM cells and treated or not with spironolactone (marked with an arrow) 2 weeks after transplantation.

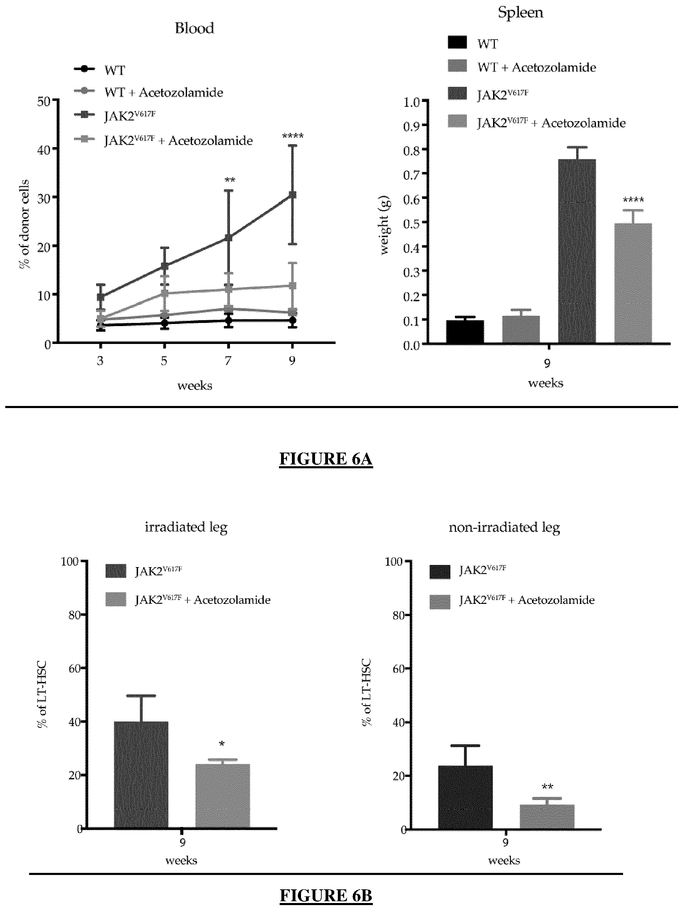

**Fig. 6****. Inhibition of carbonic anhydrase 1 by another inhibitor, Acetozolamide, also suppresses Polycythemia Vera progression.** Unless specified otherwise, the legend lists from up to bottom the corresponding entries wich appear from left to right on the graph. Kinetics of blood cells chimerisms of mice grafted with JAK2^{V617F} or WT BM cells and treated or not with Acetozolamide 2 weeks after transplantation.. **(6a. left panel).** Effects of Acetozolamide treatment starting 2 weeks after transplantation of JAK2^{V617F} or WT BM cells on spleen weights 8 weeks later. **(6a - right panel).** JAK2^{V617F} cells contribution to the LT-HSC compartments of irradiated **(6b - left panel)** and non-irradiated **(6b - right panel)** legs with or without Acetozolamide treatment starting two weeks after transplantation of JAK2^{V617F} BM cells. These contributions were measured 8 weeks later. Effect of Acetozolamide after JAK2^{V617F} transplantation marked by asterisks for statistical relevancy.

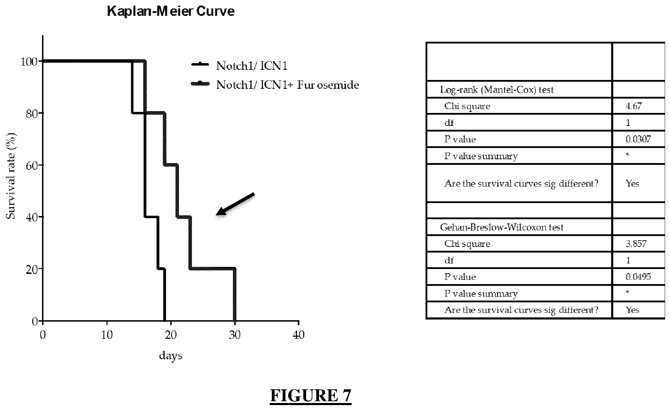

**Fig. 7****. Slowing *in vivo* leukemia progression of ICN1 leukemic cells after treatment with furosemide.** Survival curve of recipient mice grafted with 1.5x10⁴ ICN1 leukemic cells and daily treated or not with furosemide 1 week after transplantation i.e. when leukemic blast cells were detected in peripheral blood **(left panel),** Table of statistic tests performed on survival curve **(right panel).** Survival curve corresponding to furosemide treatment marked by an arrow.

### DETAILED DESCRIPTION

We have developed a novel *in vivo* approach that allows examination of the temporal progress of a mouse model of Polycythemia Vera (PV) and that can be used for any mouse models of hematopoietic malignancy. Local irradiation has been shown to alter the dynamics of hematopoietic stem cells (HSC) homeostasis by increasing their homing to the site of irradiation with almost no HSC homed in the non-irradiated leg, but this approach has never been used to follow myeloproliferative or leukemia development.

More particularly, using knock-in JAK2^{V617F} hematopoietic cells, we describe a method that allows to follow the development of hematopoietic malignancies, such as Polycythemia Vera (PV), from a unique anatomical site of immuno-competent mice and its use to characterize a new therapy.

In particular, we provide herein a new experimental *in vivo* approach to study the progression of MPN from a single irradiated anatomical site to the entire hematopoietic system and in so doing, uncover carbonic anhydrase 1 (carl) as a major contributor to Polycythemia Vera (PV).

Genetic or pharmacological inhibition of carl resulted, in this study, to increased survival associated with normalization of blood parameters of PV and of JAK2^{V617F} burden without any detrimental effect on normal hematopoiesis. As the Car1 inhibitor furosemide not only impaired PV development but could also target JAK2^{V617F} LT-HSC, our data support that this approach is suitable to stop disease progression and deplete MPN stem cells.

A second carbonic anhydrase 1 inhibitor, Acetozolamide, previously unknown as an active agent against myeloproliferative disorders, was also shown to possess therapeutic properties in another independent experiment.

Therefore, specific carl inhibitors are now shown to be potent new therapeutic drugs of JAK2^{V617F} myeloproliferative neoplasms (MPN).

To our knowledge, although the role of carbonic anhydrase in myeloid disorders had been speculated in one instance by Bonapace et al. ("Cytosolic carbonic anhydrase activity in chronic myeloid disorders with different clinical phenotype"; Biochimica et Biophysica Acta 1689 (2004) 179-181), this is the first study supporting the specific role of CA1 *in vivo* as a therapeutic target and as a biomarker.

### General definitions

As used herein, the term « *myeloproliferative disorder »* or « *myeloproliferative neoplasm* » refers to the group of hematopoietic conditions that cause an overproduction of blood cells in the bone marrow, including platelets, white blood cells and red blood cells. The group is distinct from lymphoproliferati ve disorders, or LPDs, which represent a distinct category of blood cancer targeting the lymphoid lineage.

In a non-exhaustive manner, the term « *myeloproliferative disorder »* may thus include those selected from: a Chronic Myeloid Leukemia or Chronic Myelogenous Leukemia (CML), a Chronic Neutrophilic Leukemia (CNL), Polycythemia Vera (PV), a Primary Myelofibrosis (PMF), an Essential Thrombocythemia (ET), a Chronic Eosinophilic Leukemia (CEL). The conditions reported in the Art as acute myeloid leukemia (AML) and primary or secondary myelofibrosis are further considered as myeloid disorders which may be caused or correlated to myeloproliferative disorders in the sense of the present invention.

The hematopoietic malignancies, in particular the myeloproliferative disorders or related conditions such as myeloid leukemia which are particularly considered by the invention include those associated with a JAK2 (Janus Kinase 2) somatic mutation.

As used herein, the term « *carbonic anhydrase »* or « *CA* » refers an enzyme responsible for catalyzing the reaction between carbon dioxide and water into carbonic acid and then bicarbonate, as determined by the enzyme classification EC 4.2.1.1. For the purpose of the present invention, the term « *carbonic anhydrase* » may be considered as a synonym to a « *carbonic dehydratase »,* and « *carbonic acid hydro-lyase (carbon-dioxide-forming) ».* Unless specified otherwise, the term « *carbonic anhydrase* » may thus encompass any of the reported CA families, more particularly those CA enzymes found in mammals, which includes cytosolic CAs, mitochondrial CAs, secreted CAs and membrane-associated CAs. Examples of carbonic anhydrase isoforms include those selected from the list consisting of : carbonic anhydrase 1 (CA1), carbonic anhydrase 2 (CA2), carbonic anhydrase 3 (CA3), carbonic anhydrase 4 (CA4), carbonic anhydrase 5A (CA5A), carbonic anhydrase 5B (CA5B), carbonic anhydrase 6 (CA6), carbonic anhydrase 7 (CA7), carbonic anhydrase 8 (CA8), carbonic anhydrase 9 (CA9), carbonic anhydrase 10 (CA10), carbonic anhydrase 11 (CA11), carbonic anhydrase 12 (CA12), carbonic anhydrase 13 (CA13), carbonic anhydrase 14 (CA14), carbonic anhydrase 15 (CA15).

As used herein, the term « *carbonic anhydrase 1»,* or « *CA1 », or « CA-1 » or « Carl* », refers to the corresponding isoform which is encoded in human by the CA1 gene, which corresponds to a cytosolic protein that is expressed and/or detectable in a selection of cell types, including erythrocytes.

As used herein, the term « *carbonic anhydrase activity* » refers to the catalysis, by a carbonic anhydrase, or any of its isoforms, of the catalysis of the reaction between carbon dioxide and water into carbonic acid and then bicarbonate.

As used herein, the term *« carbonic anhydrase inhibitor »* refers to any compound capable of reducing the level of expression or activity of carbonic anhydrase, or any of its isoforms. Accordingly, the term « *carbonic anhydrase inhibitor* » refers to any compound capable of reducing the level of expression or activity of carbonic anhydrase, or any of its isoforms, as determined by the enzyme classification EC 4.2.1.1.

In a non-exhaustive manner, carbonic anhydrase inhibitors are reported in Supuran ("Carbonic anhydrases: novel therapeutic applications for inhibitors and activators"; Nat Rev Drug Discov. 7(2): 168-81; 2008) and Supuran ("How many carbonic anhydrase inhibition mechanisms exist?" J. Enzyme Inhib. Med. Chem. 31, 345-360 (2016)).

It will be readily understood that, due to the number of identified isoforms of carbonic anhydrases, and unless specified otherwise, a carbonic anhydrase inhibitor may have inhibitory properties toward more than one type of isoforms.

More particularly, the term « *carbonic anhydrase 1 inhibitor* » refers to the subset of carbonic anhydrase inhibitors which are capable of reducing the level of expression or activity of, at least, carbonic anhydrase 1; in particular, including those which are capable of selectively reducing the level of expression or activity of carbonic anhydrase 1, when compared to other isoforms of carbonic anhydrases, such as those which are capable of selectively reducing the level of expression of carbonic anhydrase 1 (e.g. of selectively reducing the level of expression of *CAR1* gene or transcripts thereof) when compared to other isoforms of carbonic anhydrases.

According to some embodiments, the « *carbonic anhydrase 1 inhibitor* » may refer to a compound capable of reducing the level of expression of carbonic anhydrase 1. According to some other (not mutually-exclusive) embodiments, the « *carbonic anhydrase 1 inhibitor* » may refer to a compound capable of reducing the activity of carbonic anhydrase 1.

Exemplified CA 1 inhibitors which are particularly considered, according to the invention, include sulphonamides, in particular aromatic sulfonamide and disulfonamide derivatives, and pharmaceutically acceptable salts thereof; more particularly furosemide, acetazolamide, derivatives thereof, and their pharmaceutically acceptable salts.

Sulphonamides refer to a class of compounds comprising at least one sulfonamide functional group (RSO₂NH⁻ and RSO₂NH₂). Unless instructed otherwise, the term further encompasses secondary and tertiary sulphonamides. Sulphonamides which are particularly considered herein as carbonic anhydrase 1 inhibitors are those in which the sulfonamide functional group acts as a zinc-binding group.

Sulphonamides, aromatic sulfonamides, disulfonamides and derivatives thereof which are particularly considered, in the sense of the invention, as carbonic anhydrase 1 inhibitors, include small compounds; in particular those with an average molecular mass equal or inferior to about 1000 Dalton; more particularly those with an average molecular mass equal or inferior to about 900, 800, 700, 600 or 500 Dalton.

As used herein, the term *« furosemide »* refers to the 4-chloro-2-(furan-2-ylmethylamino)-5-sulfamoylbenzoic acid compound (IUPAC Name), or any of its pharmaceutically administrable forms, such as those sold under the name Lasix^{®}, and corresponding to the following developed formula:

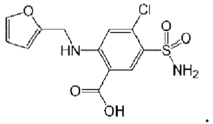

As used herein, the term « *acetazolamide »* refers to *the N*-(5-sulfainoyl-1,3,4-thiadiazol-2-yl) acetamide compound (IUPAC Name), or any of its pharmaceutically administrable forms, such as those sold under the name Diamox^{®}, and corresponding to the following developed formula:

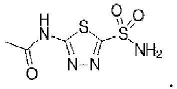

As used herein, the term « *derivative of furosemide* » and « *derivative of acetazolamide* » refers to any modified form of the furosemide or acetazolamide which retains its carbonic anhydrase 1 inhibition properties, in particular sulphonamide derivatives, such as aromatic sulfonamides and/or disulfonamides and/or those sulphonamide derivatives which include a primary, secondary or tertiary sulfonamide functional group.

In particular, the sulphonamide derivatives which are particularly considered as CA1 inhibitors, in the context of the present disclosure, are those selected from: methazolamide, ethoxzolamide, dichlorphenamide, dorzolamide, brinzolamide, sulpiride, zonisamide, topiramate, saccharin, celecoxib, hydrochlorothioazide, bumethanide,; as reported in Supuran ("How many carbonic anhydrase inhibition mechanisms exist?" J. Enzyme Inhib. Med. Chem. 31, 345-360 (2016)). Other sulphonamide derivatives which are considered as part of the carbonic anhydrase I inhibitors and which are particularly considered include clofenamide.

As used herein, *"treating"* means any manner in which one or more of the symptoms of a disease or disorder are ameliorated or otherwise beneficially altered. As used herein, amelioration of the symptoms of a particular disorder refers to any lessening of the symptoms, whether permanent or temporary, lasting or transient, that can be attributed to or associated with treatment by the compositions and methods of the present invention. Accordingly, the expression *"treating"* may include *"reversing partially or totally the effect"* of a given condition, or even *"curing"* when permanent reversal is considered.

As used herein, *"preventing"* encompasses *"reducing the likelihood of occurrence"* and *"reducing the likelihood of re-occurrence".*

The terms *"effective amount"* and *"effective to treat,"* as used herein, refer to an amount or a concentration of one or more of the compositions described herein utilized for a period of time (including acute or chronic administration and periodic or continuous administration) that is effective within the context of its administration for causing an intended effect or physiological outcome.

As used herein, a *"diagnosis"* may also encompass the *"follow-up"* of a given patient or population of patients over time.

As used herein, the singular form *"a", "an"* and *"the"* include plural references unless the context clearly dictates otherwise. For example, the term *"a pharmaceutically acceptable carrier"* encompasses a plurality of pharmaceutically acceptable carriers, including mixtures thereof.

As used herein, the expression *"at least one"* also includes « one », or « *more than one* », or « *a plurality ».*

As used herein, « *a plurality of»* may thus include « *two* » or « *two or more ».*

As used herein, « *comprising»* may include « *consisting of ».*

As used herein, a *"pharmaceutically acceptable carrier"* is intended to include any and all carrier (such as any solvents, dispersion media, coatings, antibacterial and antifungal agents, isotonic and absorption delaying agents, and the like) which is compatible with pharmaceutical administration. The use of such media and agents for pharmaceutically active substances are known. Except insofar as any conventional media or agent is incompatible with the active compound, such media can be used in the compositions of the invention.

As used herein, the term *"pharmaceutically acceptable salt"* refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge et al., describe pharmaceutically acceptable salts in detail in J. Pharmaceutical Sciences, 1977, 66, 1-19, incorporated herein by reference. Pharmaceutically acceptable salts of the compounds of this invention include those derived from suitable inorganic and organic acids and bases.

Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and N⁺(C₁₋₄alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate.

As used herein, the term « *compound capable of reducing the level of expression or activity of carbonic anhydrase* », or « *compound capable of down-regulating the level of expression of carbonic anhydrase* » as in « *compound capable of reducing the level of expression or activity of carbonic anhydrase 1* », may encompass small compounds, polypeptides and nucleic acids.

As used herein, the expression « *nucleic acid* » or « *nucleic acid molecule* » may include any form of nucleic acid that is suitable for purification in a sample, which includes, in a non-exclusive manner, DNA and RNA, in particular genomic DNA, plasmid DNA, and also PCR fragments, cDNA, mRNA, miRNA, siRNA, and also oligonucleotides and modified nucleic acids such as, for example, PMA or LMA. It is also possible to purify viral or bacterial RNA and DNA or nucleic acids from human, animal or plant sources, and/or unmodified nucleic acids; but also non-naturally occuring and/or modified nucleic acids, such as nucleic acid molecules including nucleic acid analogues and/or DNA/RNA hybrids.

CA1 inhibitors which are also considered, according to the invention, include nucleic acids down-regulating the expression of the gene encoding carbonic anhydrase 1 (*CAR1*)*,* or a transcript thereof.

Examples of nucleic acids which down-regulate the expression of the gene encoding carbonic anhydrase 1 (*CAR1*)*,* or a transcript thereof, in biological sample (i.e. an eukaryotic cell) may be selected from the group consisting of: ribozymes, antisense nucleic acids, short interfering nucleic acids (siNA), short-interfering RNAs (siRNAs), micro-RNAs (miRNA), short hairpin RNAs (shRNA), short interfering oligonucleotide, short interfering substituted oligonucleotide, short interfering modified oligonucleotide. More preferably, the compound which down-regulate the expression of the gene encoding carbonic anhydrase 1, or a fragment thereof, in an eukaryotic cell, is an interfering RNA, such as a siRNA or a miRNA; and most preferably a siRNA.

The term *"siRNA* ", or *"small interfering RNA"* is known in the Art and generally refers to small double-stranded RNAs of about 20 to 25 nucleotides, which encompasses 20, 21, 22, 23, 24 or 25 nucleotides.

The term *"miRNA* ", or *"micro-interfering RNA"* is known in the Art and generally refers to small single-stranded RNAs of about 19 to 25 nucleotides, which encompasses 19, 20, 21, 22, 23, 24 or 25 nucleotides.

In particular, examples of nucleic acids which down-regulate the expression of the gene encoding carbonic anhydrase 1 (*CAR1*)*,* or a transcript thereof, may be characterized in that they are complementary at least in part to a reference nucleic acid sequence coding for carbonic anhydrase 1; in particular in that comprise or consist of a nucleic acid sequence having sequence identity with a reference nucleic acid sequence coding for carbonic anhydrase 1 (e.g. a pre-messenger or messenger RNA coding for carbonic anhydrase 1).

As used herein, *"CRISPR system"* or « *CRISPR*/*Cas system* » refers collectively to transcripts and other elements involved in the expression of or directing the activity of CRISPR-associated ("Cas") genes, including sequences encoding one or more of: a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a *"spacer"* in the context of an endogenous CRISPR system), a guide or single-guide nucleic acid (in particular a single-guide RNA (sgRNA)) or other associated sequences and transcripts from a CRISPR locus needed for targeting a genomic region of interest. Examples of *"CRISPR system"* which are considered by the invention include Type II (or "class 2") CRISPR systems such as CRISPR/Cas9 or the more recently characterized CRISPR from *Provotella* and *Francisella 1* (Cpfl) in Zetsche et al. ("Cpfl is a Single RNA-guided Endonuclease of a Class 2 CRISPR-Cas System (2015); Cell; 163, 1-13).

According to some embodiments, the compound capable of reducing the level of expression or activity of carbonic anhydrase 1 may be selected from all or part of a CRISPR-Cas system suitable for CAR1 silencing or CAR1 deletion in an eukaryotic cell; or alternatively one or more expression vector(s) suitable for expressing all or part of the CRISPR-Cas system suitable for CAR1 silencing or CAR1 deletion in the eukaryotic cell.

According to some particular embodiments, the compound capable of reducing the level of expression or activity of carbonic anhydrase 1 may be selected from all or part of a CRISPR-Cas system selected from:
a) a first regulatory element operable in a eukaryotic cell operably linked to at least one nucleotide sequence encoding a CRISPR-Cas system guide RNA that hybridizes with the target sequence, in particular the target CAR1 gene sequence; and
b) a second regulatory element operable in a eukaryotic cell operably linked to a nucleotide sequence encoding a Cas protein, in particular a Type-II Cas9 protein

According to some more particular embodiments, the compound capable of reducing the level of expression or activity of carbonic anhydrase 1 may be selected from a guide RNA (gRNA) that hybridizes with the target CAR1 gene sequence.

Within the scope of the present invention, the term *"complementary"* is intended to mean that a first nucleic acid is complementary to a second nucleic acid when these nucleic acids have the base on each position which is the complementary (i.e. A to T, C to G) and in the reverse order. For example, the complementary sequence to TTAC is GTAA.

Within the scope of the present invention, the *"percentage identity"* between two sequences of nucleic acids or proteins means the percentage of identical nucleotides or amino acid residues between the two sequences to be compared, obtained after optimal alignment, this percentage being purely statistical and the differences between the two sequences being distributed randomly along their length. The comparison of sequences is traditionally carried out by comparing the sequences after having optimally aligned them, said comparison being able to be conducted by segment or by using an "alignment window". Optimal alignment of the sequences for comparison can be carried out, in addition to comparison by hand, by means of the local homology algorithm of Smith and Waterman (1981), by means of the local homology algorithm of Neddleman and Wunsch (1970), by means of the similarity search method of Pearson and Lipman (1988) or by means of computer software using these algorithms (GAP, BESTFIT, FASTA and TFASTA in the Wisconsin Genetics Software Package, Genetics Computer Group, 575 Science Dr., Madison, WI, or by the comparison software BLAST NR or BLAST P).

The percentage identity between two sequences is determined by comparing the two optimally-aligned sequences in which the sequence to compare can have additions or deletions compared to the reference sequence for optimal alignment between the two sequences. Percentage identity is calculated by determining the number of positions at which the nucleotide or amino acid residue is identical between the two sequences, preferably between the two complete sequences, dividing the number of identical positions by the total number of positions in the alignment window and multiplying the result by 100 to obtain the percentage identity between the two sequences. The corresponding alignment window may thus consist of the entire sequence of the nucleic acid which is considered, or a part of it (e.g. an alignment window of 15 nucleotides or more, in particular of 20 nucleotides or more).

As used herein, the term *"nucleic acid sequence identity"* may thus refer to a nucleic acid having 80% of sequence identity with a reference nucleic acid, or having more than 80% of sequence identity with the reference nucleic acid; which may thus include 80%, 81%, 82%, 83%, 84%, 85%, 86%, 87%, 88%, 89%, 90%, 91%, 92%, 93%, 94%, 95%, 96%, 97%, 98%, 99% and 100% of sequence identity with the reference nucleic acid (e.g. a nucleic acid sequence coding for carbonic anhydrase 1, such as a pre-messenger or messenger RNA).

As used herein, a *"sample"* may refer to any sample, especially to any biological sample, susceptible to contain nucleic acids. This may, in particular, encompass any sample comprising or consisting of a cell, a cell lysate, and/or a biological fluid. In a non-exclusive manner, a sample may thus consist of a cell or cell lysate, or lysate thereof. Such samples can thus be, but are not limited to, body fluid (e.g., blood, blood plasma or serum), organs, tissues, fractions, and cells isolated from mammals including, humans. In some embodiments, the biological sample is mammalian (e.g., rat, mouse, cow, dog, donkey, guinea pig, or rabbit). In certain embodiments, the biological sample is of primate origin (e.g., example, chimpanzee, or human).

### Carbonic anhydrase 1 inhibitors, and medical uses thereof

According to a **first main embodiment,** the invention relates to a carbonic Anhydrase 1 (CA1) inhibitor; for use in a method for treating or preventing a myeloproliferative disorder, an acute myeloid leukemia (AML) and/or a primary or secondary myelofibrosis.

Hence, the invention also relates to the use of a carbonic Anhydrase 1 (CA1) inhibitor for the preparation of a medicament for treating or preventing a myeloproliferative disorder, an acute myeloid leukemia (AML) and/or a primary or secondary myelofibrosis.

According to a **second main embodiment,** the invention relates to a pharmaceutical composition comprising a Carbonic Anhydrase 1 (CA1) inhibitor; for use in a method for treating or preventing a myeloproliferative disorder, an acute myeloid leukemia (AML) and/or a primary or secondary myelofibrosis. According to said embodiment, the pharmaceutical composition may comprise or consist of the Carbonic Anhydrase 1 (CA1) inhibitor in combination with a pharmaceutically acceptable carrier.

The Carbonic Anhydrase 1 (CA1) inhibitor, or pharmaceutically acceptable salt thereof, is present in the pharmaceutical composition in an effective amount, depending on the hematopoietic disorder and individual in need thereof, or population of individuals, which is to be considered.

In particular, the invention relates to the carbonic anhydrase 1 (CA1) inhibitor, or a pharmaceutical composition thereof; for use in a method as previously described; wherein the CA1 inhibitor is a sulphonamide or a pharmaceutically acceptable salt thereof.

In particular, the invention relates to the carbonic anhydrase 1 (CA1) inhibitor, or a pharmaceutical composition thereof; for use in a method as previously described; wherein the CA1 inhibitor is a sulphonamide or a pharmaceutically acceptable salt thereof, having a molecular mass equal or inferior to about 1000 Daltons (Da).

In particular, the invention relates to the carbonic anhydrase 1 (CA1) inhibitor, or a pharmaceutical composition thereof; for use in a method as previously described; wherein the CA1 inhibitor is a sulphonamide selected from the group consisting of: methazolamide, ethoxzolamide, dichlorphenamide, dorzolamide, brinzolamide, sulpiride, zonisamide, topiramate, saccharin, celecoxib, hydrochlorothioazide, bumethanide, clofenamide; or a pharmaceutically acceptable salt thereof.

In particular, the invention relates to the carbonic anhydrase 1 (CA1) inhibitor, or a pharmaceutical composition thereof; for use in a method as previously described; wherein the CA1 inhibitor is selected from Furosemide, Acetazolamide, derivatives and pharmaceutically acceptable salts thereof.

In particular, the invention relates to the carbonic anhydrase 1 (CA1) inhibitor, or a pharmaceutical composition thereof; for use in a method as previously described; wherein the CA1 inhibitor is selected from Furosemide, Acetazolamide, and pharmaceutically acceptable salts thereof.

According to some embodiments, the invention relates to the carbonic anhydrase 1 (CA1) inhibitor, or a pharmaceutical composition thereof; for use in a method as previously described; wherein the CA1 inhibitor is selected from Furosemide and pharmaceutically acceptable salts thereof.

According to some embodiments, the invention relates to the carbonic anhydrase 1 (CA1) inhibitor, or a pharmaceutical composition thereof; for use in a method as previously described; wherein the CA1 inhibitor is selected from Acetazolamide and pharmaceutically acceptable salts thereof.

Alternatively, the invention relates to the carbonic anhydrase 1 (CA1) inhibitor, or a pharmaceutical composition thereof; for use in a method as previously described; wherein the CA1 inhibitor is a nucleic acid which down-regulates the expression of the gene encoding carbonic anhydrase 1 (*CAR1*)*,* or a transcript of said gene such as a messenger RNA coding for carbonic anhydrase 1.

In particular, the invention relates to the carbonic anhydrase 1 (CA1) inhibitor, or a pharmaceutical composition thereof; for use in a method as previously described; wherein the CA1 inhibitor is a nucleic acid which down-regulates the expression of the gene encoding carbonic anhydrase 1 (*CAR1*)*,* or a transcript of said gene, the CA1 inhibitor being selected from: ribozymes, antisense nucleic acids, short interfering nucleic acids (siNA), short-interfering RNAs (siRNAs), micro-RNAs (miRNA), short hairpin RNAs (shRNA), short interfering oligonucleotide, short interfering substituted oligonucleotide, short interfering modified oligonucleotide; in particular an interfering RNA, such as a siRNA or a miRNA; and most preferably a siRNA.

According to some embodiments, the invention relates to the carbonic anhydrase 1 (CA1) inhibitor, or a pharmaceutical composition thereof; for use in a method as previously described; wherein the CA1 inhibitor is a nucleic acid having a sequence which is complementary to a nucleic acid coding for carbonic anhydrase 1, and in particular which possesses nucleic acid sequence identity with the complementary sequence of a pre-messenger or messenger RNA coding for carbonic anhydrase 1.

According to some more particular embodiments, the invention relates to the carbonic anhydrase 1 (CA1) inhibitor, or a pharmaceutical composition thereof; for use in a method as previously described; wherein the CA1 inhibitor is a nucleic acid having at least 80% nucleic acid sequence identity with a sequence which is complementary to a nucleic acid coding for carbonic anhydrase 1, and in particular is a nucleic acid having at least 80% nucleic acid sequence identity with the complementary sequence of a pre-messenger or messenger RNA coding for carbonic anhydrase 1; which includes 80%, 81%, 82%, 83%, 84%, 85%, 86%, 87%, 88%, 89%, 90%, 91%, 92%, 93%, 94%, 95%, 96%, 97%, 98%, 99% or 100%.

According to some alternative embodiments, the nucleic acid which down-regulates the expression of the gene encoding carbonic anhydrase 1 *(CAR1)* or a transcript of said gene comprises or consists of a guide RNA (gRNA) that hybridizes with the target CAR1 gene sequence.

According to particular embodiments, the invention relates to the carbonic anhydrase 1 (CA1) inhibitor, or a pharmaceutical composition thereof; for use in a method for treating or preventing an hematopoietic disorder selected from: a chronic myeloid leukemia (CML), a chronic neutrophilic leukemia (CNL), polycythemia vera (PV), a primary myelofibrosis (PMF), an essential thrombocythemia (ET), a chronic eosinophilic leukemia

According to more particular embodiments, the invention relates to the carbonic anhydrase 1 (CA1) inhibitor, or a pharmaceutical composition thereof; for use in a method for treating or preventing an hematopoietic disorder selected from: a chronic myeloid leukemia (CML), a chronic neutrophilic leukemia (CNL), polycythemia vera (PV), a primary myelofibrosis (PMF), an essential thrombocythemia (ET), a chronic eosinophilic leukemia; and wherein the CA1 inhibitor is a sulphonamide or a pharmaceutically acceptable salt thereof.

According to more particular embodiments, the invention relates to the carbonic anhydrase 1 (CA1) inhibitor, or a pharmaceutical composition thereof; for use in a method for treating or preventing an hematopoietic disorder selected from: a chronic myeloid leukemia (CML), a chronic neutrophilic leukemia (CNL), polycythemia vera (PV), a primary myelofibrosis (PMF), an essential thrombocythemia (ET), a chronic eosinophilic leukemia; and wherein the CA1 inhibitor is selected from Furosemide, Acetazolamide, and pharmaceutically acceptable salts thereof.

For instance, the invention relates to Furosemide and pharmaceutically acceptable salts thereof; for use in a method for treating or preventing an hematopoietic disorder selected from: a chronic myeloid leukemia (CML), a chronic neutrophilic leukemia (CNL), polycythemia vera (PV), a primary myelofibrosis (PMF), an essential thrombocythemia (ET), a chronic eosinophilic leukemia.

For instance, the invention relates to Acetazolamide and pharmaceutically acceptable salts thereof; for use in a method for treating or preventing an hematopoietic disorder selected from: a chronic myeloid leukemia (CML), a chronic neutrophilic leukemia (CNL), polycythemia vera (PV), a primary myelofibrosis (PMF), an essential thrombocythemia (ET), a chronic eosinophilic leukemia.

According to particular embodiments, the invention relates to the carbonic anhydrase 1 (CA1) inhibitor, or a pharmaceutical composition thereof; for use in a method for treating or preventing a polycythemia vera (PV), an acute myeloid leukemia (AML), a primary myelofibrosis (PMF) and/or a secondary myelofibrosis.

According to more particular embodiments, the invention relates to the carbonic anhydrase 1 (CA1) inhibitor, or a pharmaceutical composition thereof; for use in a method for treating or preventing a polycythemia vera (PV), an acute myeloid leukemia (AML), a primary myelofibrosis (PMF) and/or a secondary myelofibrosis; and wherein the CA1 inhibitor is a sulphonamide or a pharmaceutically acceptable salts thereof.

According to more particular embodiments, the invention relates to the carbonic anhydrase 1 (CA1) inhibitor, or a pharmaceutical composition thereof; for use in a method for treating or preventing a polycythemia vera (PV), an acute myeloid leukemia (AML), a primary myelofibrosis (PMF) and/or a secondary myelofibrosis; and wherein the CA1 inhibitor is selected from Furosemide, Acetazolamide, and pharmaceutically acceptable salts thereof.

For instance, the invention relates to Furosemide and pharmaceutically acceptable salts thereof; for use in a method for treating or preventing a polycythemia vera (PV), an acute myeloid leukemia (AML), a primary myelofibrosis (PMF) and/or a secondary myelofibrosis.

For instance, the invention relates to Acetazolamide and pharmaceutically acceptable salts thereof; for use in a method for treating or preventing a polycythemia vera (PV), an acute myeloid leukemia (AML), a primary myelofibrosis (PMF) and/or a secondary myelofibrosis.

According to particular embodiments, the invention relates to the carbonic anhydrase 1 (CA1) inhibitor, or a pharmaceutical composition thereof; for use in a method for treating or preventing a primary myelofibrosis (PMF) or a secondary myelofibrosis.

According to more particular embodiments, the invention relates to the carbonic anhydrase 1 (CA1) inhibitor, or a pharmaceutical composition thereof; for use in a method for treating or preventing a primary myelofibrosis (PMF) or a secondary myelofibrosis; and wherein the CA1 inhibitor is a sulphonamide or a pharmaceutically acceptable salts thereof.

According to more particular embodiments, the invention relates to the carbonic anhydrase 1 (CA1) inhibitor, or a pharmaceutical composition thereof; for use in a method for treating or preventing a primary myelofibrosis (PMF) or a secondary myelofibrosis; and wherein the CA1 inhibitor is selected from Furosemide, Acetazolamide, and pharmaceutically acceptable salts thereof.

For instance, the invention relates to Furosemide and pharmaceutically acceptable salts thereof; for use in a method for treating or preventing a primary myelofibrosis (PMF) or a secondary myelofibrosis.

For instance, the invention relates to Acetazolamide and pharmaceutically acceptable salts thereof; for use in a method for treating or preventing a primary myelofibrosis (PMF) or a secondary myelofibrosis.

### Carbonic anhydrase 1 and its use as a biomarker

According to a **third main embodiment,** the invention relates to an *in vitro* or *ex vivo* use of a level of expression or activity of Carbonic Anhydrase 1 (CA1), as a biomarker of a myeloproliferative disorder, an acute myeloid leukemia (AML) and/or a primary or secondary myelofibrosis.

According to a particular embodiment, the invention relates to an *in vitro* or *ex vivo* use of a level of expression of Carbonic Anhydrase 1 (CA1), as a biomarker of a myeloproliferative disorder, an acute myeloid leukemia (AML) and/or a primary or secondary myelofibrosis.

According to a particular embodiment, the invention relates to an *in vitro* or *ex vivo* use of a level of activity of Carbonic Anhydrase 1 (CA1), as a biomarker of a myeloproliferative disorder, an acute myeloid leukemia (AML) and/or a primary or secondary myelofibrosis.

According to a **fourth main embodiment,** the invention relates to an *in vitro* or *ex vivo* method for the diagnosis or follow-up of a myeloproliferative disorder, an acute myeloid leukemia (AML) and/or a primary or secondary myelofibrosis in an individual, comprising the steps of:
a) determining a level of expression or activity of Carbonic Anhydrase 1 (CA1) in a biological sample from the individual;
b) comparing the level of expression or activity of CA1 to a reference value, wherein an **increase** of the level of expression or activity of Carbonic Anhydrase 1 (CA1) is indicative of the occurrence of said myeloproliferative disorder, acute myeloid leukemia (AML) and/or primary or secondary myelofibrosis in the individual.

According to a particular embodiment, the invention relates to an *in vitro* or *ex vivo* method for the diagnosis or follow-up of a myeloproliferative disorder, an acute myeloid leukemia (AML) and/or a primary or secondary myelofibrosis in an individual, comprising the steps of:
a) determining a level of expression of Carbonic Anhydrase 1 (CA1) in a biological sample from the individual;
b) comparing the level of expression of CA1 to a reference value, wherein an **increase** of the level of expression of Carbonic Anhydrase 1 (CA1) is indicative of the occurrence of said myeloproliferative disorder, acute myeloid leukemia (AML) and/or primary or secondary myelofibrosis in the individual.

According to a particular embodiment, the invention relates to an *in vitro* or *ex vivo* method for the diagnosis or follow-up of a myeloproliferative disorder, an acute myeloid leukemia (AML) and/or a primary or secondary myelofibrosis in an individual, comprising the steps of:
a) determining a level of activity of Carbonic Anhydrase 1 (CA1) in a biological sample from the individual;
b) comparing the level of activity of CA1 to a reference value, wherein an **increase** of the level of activity of Carbonic Anhydrase 1 (CA1) is indicative of the occurrence of said myeloproliferative disorder, acute myeloid leukemia (AML) and/or primary or secondary myelofibrosis in the individual.

According to a **fifth main embodiment,** the invention relates to an *in vitro* or *ex vivo* method for screening an active agent for the treatment or prevention of a myeloproliferative disorder, an acute myeloid leukemia (AML) and/or a primary or secondary myelofibrosis, comprising the steps of:
a) determining a level of expression or activity of Carbonic Anhydrase 1 (CA1) in a biological sample;
b) comparing the level of expression or activity of CA1 to a reference value, wherein a **decrease** of the level of expression or activity of Carbonic Anhydrase 1 (CA1) in the biological sample by the active agent is indicative of its efficacy for the treatment or prevention of said myeloproliferative disorder, acute myeloid leukemia (AML) and/or primary or secondary myelofibrosis.

According to a particular embodiment, the invention relates to an *in vitro* or *ex vivo* method for screening an active agent for the treatment or prevention of a myeloproliferative disorder, an acute myeloid leukemia (AML) and/or a primary or secondary myelofibrosis, comprising the steps of:
a) determining a level of expression of Carbonic Anhydrase 1 (CA1) in a biological sample;
b) comparing the level of expression of CA1 to a reference value, wherein a **decrease** of the level of expression of Carbonic Anhydrase 1 (CA1) in the biological sample by the active agent is indicative of its efficacy for the treatment or prevention of said myeloproliferative disorder, acute myeloid leukemia (AML) and/or primary or secondary myelofibrosis.

According to a particular embodiment, the invention relates to an *in vitro* or *ex vivo* method for screening an active agent for the treatment or prevention of a myeloproliferative disorder, an acute myeloid leukemia (AML) and/or a primary or secondary myelofibrosis, comprising the steps of:
a) determining a level of activity of Carbonic Anhydrase 1 (CA1) in a biological sample;
b) comparing the level of activity of CA1 to a reference value, wherein a **decrease** of the level of activity of Carbonic Anhydrase 1 (CA1) in the biological sample by the active agent is indicative of its efficacy for the treatment or prevention of said myeloproliferative disorder, acute myeloid leukemia (AML) and/or primary or secondary myelofibrosis.

According to some embodiments, the above-mentioned methods may further include a step of determining a level of expression or activity of a Carbonic Anhydrase in the biological sample which is different from Carbonic Anhydrase 1.

According to said particular embodiments, the method may further incldue a step of comparing the level of expression or activity of one or more Carbonic Anhydrase isoforms distinct from Carbonic Anhydrase 1, to the corresponding level of expression or activity of Carbonic Anhydrase 1 in the biological sample; thereby determining the level of expression or activity of CA1 which is modulated by the active agent.

For instance, the level of expression or activity of CA1 may be compared to the level of expression or activity of one or more carbonic anhydrase isoforms selected from: carbonic anhydrase 2 (CA2), carbonic anhydrase 3 (CA3), carbonic anhydrase 4 (CA4), carbonic anhydrase 5A (CA5A), carbonic anhydrase 5B (CA5B), carbonic anhydrase 6 (CA6), carbonic anhydrase 7 (CA7), carbonic anhydrase 8 (CA8), carbonic anhydrase 9 (CA9), carbonic anhydrase 10 (CA10), carbonic anhydrase 11 (CA11), carbonic anhydrase 12 (CA12), carbonic anhydrase 13 (CA13), carbonic anhydrase 14 (CA14), carbonic anhydrase 15 (CA15).

In a non-limitative manner, methods for determining the level of activity of carbonic anhydrase inhibitors is reported in Supuran ("Carbonic anhydrases: novel therapeutic applications for inhibitors and activators"; Nat Rev Drug Discov. 7(2):168-81; 2008) and Supuran ("How many carbonic anhydrase inhibition mechanisms exist?" J. Enzyme Inhib. Med. Chem. 31, 345-360 (2016)).

According to particular embodiments of the **fourth** and **fifth main embodiment,** the biological sample is a blood sample that comprises CD34-positive cells.

Examples of CD34-positive cells include those cells or tissues selected from the group consisting of: alveolar soft part sarcoma, preB-ALL, acute myeloid leukemia (AML), myeloproliferative disorder, primary or secondary myelofibrosis AML-M7, dermatofibrosarcoma protuberans, gastrointestinal stromal tumors, giant cell fibroblastoma, granulocytic sarcoma, Kaposi's sarcoma, liposarcoma, malignant fibrous histiocytoma, malignant peripheral nerve sheath tumors, meningeal hemangiopericytomas, meningiomas, neurofibromas, schwannomas, and papillary thyroid carcinoma.

Other non-mutually-exclusive examples of CD34-positive cells may include CD34-positive stem cells, such as CD34-positive hematopoietic stem cells.

Accordingly the above-mentioned methods may further comprise a step of providing a biological sample, such as a biological sample comprising or consisting of CD34-positive cells.

The method for the diagnosis or follow-up may, in particular, be applied for the follow-up of an individual or group of individual, including compliance or efficacy of a treatment by an active agent. Accordingly, the method may advantageously comprise a step of comparing the level of expression or activity of Carbonic Anhydrase 1 (CA1) in a biological sample of an individual, which was previously obtained before and after administration of an active agent to the said individual.

In that context, the expression *"compliance or efficacy of a treatment by an active agent"* may also refer to a change in the posology or dosage of an active agent, or alternatively of a combination of active agents.

According to particular embodiments of the **fourth and fifth main embodiment,** the active agent is selected from sulphonamides, pharmaceutically acceptable salts and derivatives thereof; in particular a sulphonamide selected from the group consisting of: methazolamide, ethoxzolamide, dichlorphenamide, dorzolamide, brinzolamide, sulpiride, zonisamide, topiramate, saccharin, celecoxib, hydrochlorothioazide, bumethanide, clofenamide, and pharmaceutically acceptable salts thereof; more particularly Furosemide, Acetazolamide, and pharmaceutically acceptable salts thereof.

### EXAMPLES

### Material & Methods

**Mice.** The conditional flexed Jak2^{V617F} KI mice **(**Hasan et al. ; Blood 122, 1464-1477 ; 2013) were crossed with VavCre transgenic (TG) mice (Croker et al., SOCS3 Is a Critical Physiological Negative Regulator of G-CSF Signaling and Emergency Granulopoiesis. Immunity 20, 153-165 (2004)) to express the mutant *Jak2.* To easily evaluate allele burden in kinetic experiments, Jak2^{V617F} KI/VavCre mice were also crossed with *UbiGFP* TG mice in Gustave Roussy' animal facilities. For one-leg transplantation, F1 crossed B6.SJL/BoyJ (CD45.1) x C57BL/6J (CD45.2) mice were used as recipient mice. All mice were housed in a specific pathogen-free section at an animal facility of CEA/DRF/IBFJ/iRCM according to the institutional guidelines and French Ministry of Agriculture's regulations.

**One leg irradiation and transplantation.** Recipient mice were anesthetized with ketamine-xylazine and their leg was fixed on a tray with only the leg left towards the opposite side to their body. The tray was put in the radiation field of a GSRD1 radiation equipment. The mouse body was shielded from irradiation with lead plates and the leg was irradiated at 2x 5Gy with an interval of 17h. Bone marrow (BM) cells (1 × 10⁷) were intravenously transplanted into the irradiated mice 7 hours after the second irradiation. For barcode and shRNA experiments, 10,000 transduced Lir⁻Sca1⁺cKit⁺ (LSK) hematopoietic cells were intrafemorally transplanted into irradiated leg under Isoflurane anesthetic.

**Blood analysis.** Blood was collected and analyzed through a hemocytometer (CD3700, ABBOTT). The rest of blood was subjected to hemolysis by using an ammonium chloride solution (STEMCELL). The hemolyzed blood were stained with antibodies against CD45.1 (A20) and CD45.2 (104) and analyzed through flow cytometry to examine blood chimerism.

**Flow cytometry analysis.** Recipient mice were sacrificed at the indicated time. BM cells (femur and tibia) of irradiated leg and of non-irradiated leg were harvested by flushing with 1 mL of PBS. After centrifugation, BM supernatants were collected and kept at -80°C with anti-protease (ROCHE) for chemokine analyses. BM cells were suspended in PBS and splenocytes were also collected in PBS by crushing spleen on a 70 µm filter. Before hemolysis, cells were stained with Ter119 (TER-119) and CD71 (C2) antibodies for the examination of erythroid compartments. The rest of cells were hemolyzed and stained for analyses of granulocytes, hematopoietic progenitor cells (HPCs) and hematopoietic stem cells (HSCs) by using antibodies against CD45.1 (A20), CD45.2 (104), CD11b (M1/70), Gr-1 (RB6-8C5), c-Kit (2B6), Sca-1 (D7 or E13-161.7), CD34 (RAM34 or MEC14.7), CD16/32 (93), CD48 (HM48-1) and CD150 (TC15-12F12.2). For HPCs and HSCs, cells were stained with Lineage Cell Detection Cocktail-Biotin (Miltenyi Biotec) revealed with PE-CF594 Streptavidin antibody. For cell cycle analyses, stained HSPCs were permeabilized with Cytofix/Cytoperm buffer following manufacturer's instructions (BD Biosciences) and labelled with anti-Ki67 antibody (SolA15) and Hoechst 33342. All antibodies were purchased from BioLegend, eBioscience, or BD Biosciences. Flow cytometry analyses were performed through FACS Canto II (BD Biosciences) and LSR II (BD Biosciences), and cell sorting was conducted by using FACS Aria II (BD Biosciences). Analyses were performed with the FlowJo software (TRISTAR) and illustrated with the Prism7 software (GraphPad).

**Colony assay.** One hundred microliter of blood was collected from recipient mice and hemolyzed by an ammonium chloride solution (STEMCELL). The hemolyzed cells were plated in 3 ml of MethoCult GF M3434 (STEMCELL) on a 35 mm dishes. The number of total and donor GFP⁺ colonies were counted at 14 days on an EVOS FL Auto Microscope (ThermoFisher).

**Chemokines assay.** Chemokines of BM supernatant from irradiated and non-irradiated leg of recipient mice were analyzed with a proteome profiler mouse XL cytokine array following manufacturer's instructions (BIOTECHNE). Signal quantification was done with ImageQuant TL software (GE Helathcare).

**Barcode experiment.** 1 × 10⁵ Donor WT or JAK2^{V617F} LSK sorted cells were incubated with 10uL of a vector library of 2,500 barcodes in a StemSpan SFEM medium (STEMCELL) with mSCF (50 ng/ml; R&D system) at 37°C for 6 hours. After removal of virus medium, 1×10⁵ transduced LSK cells were intrafemorally transplanted into the irradiated leg of recipient mice. The transduction efficiency was in a range of 15-20%. After developing PV disease symptoms, mice were sacrificed and BM cells were separately collected from the irradiated leg and the non-irradiated leg, stained, and the donor-derived GFP⁺ cells were sorted from the indicated cell fractions. Genome DNA was extracted by lysing the cells with Proteinase K solution (Invitrogen) at 55°C for 2 hours. After the lysis, the DNA samples were split into technical duplicates. Amplification reaction, sequencing and barcode classification were performed as described in Perié L et al. (The Branching Point in Erythro-Myeloid Differentiation. Cell 163, 1655-1662 (2015)). Using a customized script in R, sample data were filtered, normalized (to 10⁵ reads), and classified by tagging each barcode. Read counts were transformed using the hyperbolic arcsine function that is similar to a logarithmic function but can accommodate barcode reads with a value of 0 as described (Cell 2015, 163:1655-1662). Resulting data have been graphed with a customized script in R from the R Graph Gallery.

**Splenectomy and secondary transplantation.** Recipient mice were irradiated on one leg, intravenously transplanted with 1×10⁷ donor WT or JAK2^{V617F} BM cells and splenectomized 1 or 2 week(s) after grafting by surgical intervention under Isoflurane anesthetic. For secondary transplantation experiments, BM cells were separately harvested from irradiated leg and non-irradiated leg of recipient mice 5 weeks after transplantation, then hemolyzed and transplanted at 1×10⁷ into secondary non-conditioned immunocompetent mice.

**Transcriptome and quantitative RT-PCR analysis.** 1 × 10⁵ Donor JAK2^{V617F} LSK sorted cells were intrafemorally transplanted into the irradiated leg of recipient mice. After development of PV disease symptoms, JAK2^{V617F} HSPCs (ST-HSC and LT-HSCs) cells from irradiated and non-irradiated leg BM were sorted and lysed with RLT Plus Buffer (QIAGEN). Total RNA was extracted according to the protocol of RNeasy Plus Micro Kit (QIAGEN). The RNA integrity (RNA Integrity Score≥7.0) was checked on the Agilent 2100 Bioanalyzer (Agilent) and quantity was determined using Qubit (Invitrogen). For transcriptome analysis, total RNA samples were prepared with the GeneChip^{™} Whole-Transcript (WT) Pico Reagent Kit and analyzed with the GeneChip^{™} Whole-Transcript Expression array using Clariom S assay following manufacturer's instructions (Thermo Fisher). Data were collected and analyzed with the TAC software recommended by the manufacturer (Thermo Fisher). For quantitative RT-PCR analysis, total RNA was extracted according to the protocol of RNeasy Plus Micro Kit (QIAGEN) and cDNA were synthesized from the RNA by the reverse transcription with SuperScript IV First-Strand Synthesis System (Invitrogen). Quantitative RT-PCR was performed by using StepOnePlus Real-Time PCR System with TaqMan Fast Advanced Master Mix (Applied Biosystems). Primers used for detection of target carbonic anhydrase 1 gene (Car1 (Mm00486717_m1) were purchased from Applied Biosystems. The gene expression level was calculated based on the 2^{-ΔΔCT} method.

***Carl* shRNA.** To perform inhibition experiments, shRNA sequences directed against MmCar1 gene have been cloned in pTRIP-MND-Cherry-H1 plasmid (from CEA/iRCM/CIGEx). Briefly, shRNA sequences were generated by annealing complementary pair of primers harboring homology sequence to pTRIP-MND-Cherry-H1 plasmid digested by *Bam*HI for further cloning by complementary single-strand annealing based method (Li M. Z. et al., Harnessing homologous recombination in vitro to generate recombinant DNA via SLIC. Nat. Methods 4, 251-256 (2007)). All enzymes are from New England Biolabs (Ipswich, MA), antibiotics from Sigma-Aldrich (St Louis, MO) and primers from Eurofins Genomics (Ebersberg, Germany) (table 1). All constructs were validated by DNA sequencing. The 1 × 10⁵ Donor WT or JAK2^{V617F} LSK sorted cells were transduced with the 3 *Car1* shRNAs or Ctl shRNA and intrafemorally transplanted into the irradiated leg of recipient mice. Over 85% of the transduced LSK cells were detected positive for fluorescence expression of Sherry.

**Table 1: oligonucleotides used**

| Name | Sequence |
|---|---|
| shRNA-MmCar1-1_F | GTTCTGTATGAGACCACAGGGGTCCCCCAGAACAGTCAGAGCCTCATTTCTCGAGAAAT GAGGCTCTGACTGTTCTTTTTT (SEQ ID N°1) |
| shRNA-MmCar1-1_R | GACCCACCTCCCAACCCCGAGAAAAAGAACAGTCAGAGCCTCATTTCTCGAGAAATGA GGCTCTGACTGTTCTGGGG (SEQ ID N°2) |
| shRNA-MmCar1-4_F | GTTCTGTATGAGACCACAGGGGTCCCCCGCTTCCTTCATCTCTGGATTACTCGAGTAAT CCAGAGATGAAGGAAGCTTTTT (SEQ ID N°3) |
| shRNA-MmCar1-4_R | GACCCACCTCCCAACCCCGAGAAAAAGCTTCCTTCATCTCTGGATTACTCGAGTAATCC AGAGATGAAGGAAGCGGGG (SEQ ID N°4) |
| shRNA-MmCar1-5_F | GTTCTGTATGAGACCACAGGGGTCCCCCCGTGGATGGAACTAGATATTCTCGAGAATA TCTAGTTCCATCCACGGTTTTT (SEQ ID N°5) |
| shRNA-MmCar1-5_R | GACCCACCTCCCAACCCCGAGAAAAACCGTGGATGGAACTAGATATTCTCGAGAATATC TAGTTCCATCCACGGGGGG (SEQ ID N°6) |

**Pharmacologic treatment.** Furosemide was purchased from Sigma (MKCF8657), dissolved in DMSO for stock solution (50mg/mL) and diluted in PBS solution prior to use. At indicated time, recipient mice were daily treated by intravenous injection of furosemide at 20mg/Kg. Spironolactone (SIGMA) was prepared in water bottles at 250mg/Kg to treat recipient mice as described **(**Rafael-Fortney et al., "Early treatment with lisinopril and spironolactone preserves cardiac and skeletal muscle in duchenne muscular dystrophy mice » ; Circulation, 2011; 124:582-8). Acetozolamide was purchased from SIGMA (A6011), dissolved in PBS and solubilized with NaOH (35%) until complete dissolution of the product. One leg irradiated recipient mice were daily treated or not by intravenous injection of acetazolamide at 200mg/Kg two weeks after transplantation of 10⁷ JAK2^{V617F} hematopoietic cells.

**ICN1 leukemia.** Non-conditioned C57BL/6J (CD45.2) recipient mice were intravenously transplanted with 1.5x10⁴ ICN1 leukemic cells in 100uL of PBS. Seven days after transplantation, when blast cells are detected in the peripheral blood, recipient mice were daily treated or not by intravenous injection of furosemide at 20mg/Kg.

**Whole-transcriptome RNA-seq on CD34+ cell from Patients.** The patients were selected for their high JAK2^{V617F} variant allele frequency (from 60 to 100% VAF). Blood samples from patients were obtained from Gustave Roussy (Villejuif, France), Saint Louis Hospital (Paris, France), Saint Antoine Hospital (Paris, France), with the agreement from the Comité de Protection des Personnes (CPP) Ile de France IV- Institutional review board (agreement from US Department of Health and Human Services (n°IRB 00003835-Protocol 2015/59-NICB) and Commission Nationale de l'Informatique et des Libertés (authorization #915663). Written informed consents were obtained in accordance with the Declaration of Helsinki. Control donor samples were obtained from donors after leukapheresis. Peripheral blood from patients was collected in EDTA tubes. Hematopoietic progenitors (CD34⁺) were isolated from mononuclear cells (MNCs) by immunomagnetic enrichment (Miltenyi, Biotech) and were amplified for 5 days in serum-free medium in the presence of a cocktail of human recombinant cytokines containing EPO (1 U/mL) (Amgen Thousand Oaks, CA), TPO (20 ng/mL) (Kirin, Japan), SCF (25 ng/mL) (Biovitrum AB, Sweden), IL-3 (10 ng/mL), FLT3-L (10 ng/mL), G-CSF (20 ng/mL) and IL-6 (100 U/mL) (MiltenyiBiotec). Total RNA was isolated using the RNeasy Mini Kit (Qiagen). The RNA integrity (RNA Integrity Score≥7.0) was checked on the Agilent 2100 Bioanalyzer (Agilent) and quantity was determined using Qubit (Invitrogen). SureSelect Automated Strand Specific RNA Library Preparation Kit was used according to manufacturer's instructions with the Bravo Platform. Briefly, 50 to 200ng of total RNA sample was used for poly-A mRNA selection using oligo(dT) beads and subjected to thermal mRNA fragmentation. The fragmented mRNA samples were subjected to cDNA synthesis and were further converted into double stranded DNA using the reagents supplied in the kit, and the resulting dsDNA was used for library preparation. The final libraries were bar-coded, purified, pooled together in equal concentrations and subjected to paired-end sequencing on Novaseq-6000 sequencer (Illumina) at Gustave Roussy.

**Automated capillary immunoassay (WES).** Automated capillary immunoassay (Simple Western) was performed on a Western immunoassay (WES) system (Protein Simple). Car1 (NBP1, BIOTECHNE) and GAPDH (Cell Signaling Technologies) antibodies were respectively used at a 1:50 and 1:100 dilution. The analyses were performed on a 12-230 kDa separation module (SM-W004) according to the manufacturer's instructions (Protein Simple).

**Statistical analyses.** Data are provided as mean ± SEM. All data were tested for significance using two-way ANOVA following post-hoc analysis. Survival outcome was investigated by Kaplan-Meier plot and tested with log-rank test statistics. In all tests, P values of less than 0.05 were considered statistically significant (*<0.05, **<0.01, ***<0.001, ****<0.0001).

### Example 1.

### A new method to follow hematopoietic malignancies and other myeloproliferative disorder progression in immuno-competent mice. Application to a model of Polycythemia Vera.

Sequential irradiation (5Gy 2x with 17hr rest) of a single C57BL/6J mouse leg resulted, 7 hours after the second irradiation, in decreased bone marrow (BM) cellularity and in decreased numbers of long-term hematopoietic stem cells (LT-HSC) only in the irradiated leg. Transplantation of wild type (WT) BM cells into the sequentially irradiated leg led to a stable but largely restricted engraftment in the irradiated leg **(****Fig. 1****).** In contrast, transplantation of JAK2^{V617F} BM cells from knock-in mice into the irradiated leg was associated with a progressive increase of JAK2^{V617F} cells into both legs **(****Fig. 1a**) without any change of BM cellularity. JAK2^{V617F} LT-HSC number was dramatically increased 6 weeks after transplantation in BM of irradiated and non-irradiated legs as did JAK2^{V617F} multi-potent progenitors (MPP) **(****Fig. 1b**) and granulocyte-monocyte progenitors (GMP) indicating differentiation of mutant LT-HSC. Of note, the number of endogenous LT-HSC remained relatively static despite the increased number of mutant LT-HCS **(****Fig. 1b**, 6 weeks). In both legs but only after transplantation of JAK2^{V617F} BM cells, the percentages of endogenous Lin⁻Sca⁺cKit⁺ (LSK) cells and megakaryocyte-erythroid progenitors (MEP) but not GMP in G₀ phase increased whereas the percentage of JAK2^{V617F} MEP in S-G2M phase increased **(****Fig. 1c**). This was associated with an expansion of JAK2^{V617F} MEP at the expense of endogenous MEP and an almost full contribution of JAK2^{V617F} cells to the immature erythroid cell compartment (CD71⁺Ter119⁺) without expansion of this compartment in both legs as early as two weeks after transplantation **(****Fig. 1d****).** Mice locally transplanted with JAK2^{V617F} BM cells in a single irradiated leg developed PV syndrome as assessed by blood parameters and progressive increased JAK2^{V617F} cell burden **(****Fig. 1e** and **If)** followed by death of 80% of mice 9 weeks' post-transplantation **(****Fig. 1g**).

**Altogether, these results indicate that, through this single irradiated-leg mouse model, JAK2^{V611F} hematopoietic cell dynamics associated with PV progression can be studied in an immuno-competent mouse model.**

### Example 2.

### JAK2^{V617F} LSK cells from the irradiated leg colonize the non-irradiated leg.

Chemokine detection assays revealed increased levels of chemokines involved in hematopoietic stem and progenitor cell (HSPC) mobilization only in the supernatant from the irradiated leg and only after JAK2^{V617F} BM engraftment. These increased levels of chemokines were associated with increased number of circulating JAK2^{V617F} HSPC starting 7 days after transplantation **(****Fig. 2a****)** suggesting that JAK2^{V617F} LSK grafted into the irradiated leg could migrate to the non-irradiated leg. To address any migration, JAK2^{V617F} LSK cells were transduced with a barcodes library (see Perié et al. ; "The Branching Point in Erythro-Myeloid Differentiation". Cell 163, 1655-1662 (2015)), at a MOI of 1 and transplanted into the irradiated-leg of recipient mice **(****Fig. 2b****).**

The PV development of these mice was slower as shown by the hematocrit kinetics and their survival curve. Analysis of bare-coded JAK2^{V617F} LSK eleven weeks after transplantation of bare-coded JAK2^{V617F} LSK showed that the number of clones found in the irradiated leg (37) was more than two fold higher than in the non-irradiated leg (15) where all except two clones detected were present in the irradiated leg. In addition, individual analysis of three mice showed that independently of the number and relative proportion of clones found in the irradiated leg, only one or two bare-coded JAK2^{V617F} LSK are highly represented in the non-irradiated leg. As for erythroid cells, 75% (irradiated leg) and 66% (non-irradiated leg) of cells shared bare-codes. Of interest, whereas one JAK2^{V617F} LSK clone was specifically found in the irradiated leg, erythroid cells derived from this clone were found in both legs indicating that this clone was either initially present in the non-irradiated leg and got exhausted or that this clone highly differentiated into erythroid/myeloid cells in the non-irradiated leg and thus could not be detected. All except one JAK2^{V617F} LSK clone present in the two legs gave rise to erythroid cells in the non-irradiated cells and most of the LSK clones detected in both legs also gave rise to myeloid cells.

**Altogether, these results show that JAK2^{V617F} Lin⁻Sca⁺cKit⁺ (LSK) clones but not WT LSK clones from the irradiated leg can colonize the non-irradiated leg and contribute to erythroid and myeloid cells production.**

### Example 3.

### The spleen is necessary for PV development through amplification of the JAK2^{V617F} erythroid compartment.

Although splenomegaly is associated with myeloproliferative neoplasms (MPN), the role of spleen in the development of these neoplasms is incompletely defined. Spleen weight/size increased after transplantation of JAK2^{V617F} hematopoietic cells **(****Fig. 3a****)** with an increased JAK2^{V617F} chimerism in spleen white blood cells. JAK2^{V617F} LSK cells and multipotent progenitors (MPP) contribution to their respective compartments increased without any significant increase of these compartments **(****Fig. 3b****).** As in BM, the megakaryocyte-erythroid progenitors (MEP) compartment increased due increased contribution of JAK2^{V617F} MEP but, in contrast to BM, this increase was associated with a very high CD71⁺Ter119⁺ cells expansion as early as two weeks after transplantation **(****Fig. 3c****)** suggesting that the spleen amplifies the JAK2^{V617F} erythroid compartment and greatly increased red blood cells production in PV. Bare-code analysis showed that more than 90% of LSK clones present in the irradiated and non-irradiated legs were also present in the spleen. As for erythroid cells, 81% of clones present in the irradiated and/or non-irradiated legs were found in the spleen that also contained 31% of clones not found in the irradiated or the non-irradiated leg. To study the role of spleen in PV development, mice were splenectomised 1 week after transplantation of JAK2^{V617F} hematopoietic cells. These splenectomised mice survived indicating escape from PV syndrome **(****Fig. 3d****-e),** showed normal blood parameters and a blood chimerism similar to the one in mice transplanted with WT cells until 16 weeks after transplantation **(****Fig. 3f, 3g****,** **3h and 3i****).** At 26 weeks, these mice showed a decreased cellularity in both legs **(****Fig. 3j****)** associated with a very high contribution of JAK2^{V617F} cells in the myeloid, erythroid compartments **(****Fig. 3k** and **3l****)** and in LT-HSC **(****Fig. 3m****)** indicating that these mice survived with an almost complete mutant cells hematopoiesis. Similar results were obtained when splenectomy was performed 2 weeks after transplantation *i.e*. when mutant hematopoiesis started to be detectable in the non-irradiated leg.

**These results indicate that amplification of JAK2^{V617} erythroid cells relies on spleen and that PV cannot develop, in this mouse model of hematopoietic malignancy, in the absence of spleen.**

### Example 4.

### Carbonic anhydrase 1 is a specific biomarker and inhibition of carbonic anhydrase 1 by Furosemide suppresses PVprogression.

Evolution and/or selection of JAK2^{V611F} clones that have migrated were functionally studied by secondary transplantation. Whereas 20% of immuno-competent mice transplanted with BM from the irradiated leg of primary recipient mice developed PV, up to 66% of mice transplanted with BM from the non-irradiated leg developed PV **(****Fig. 4a****)** indicating that the LSK that have migrated and engrafted exhibited increased fitness for a non-irradiated environment. We thus searched for the molecular basis of this increased fitness, which could overlap with the clonal dominance acquisition of mutated HSC during natural human disease. To this end, we compared transcriptomic profiling of sorted JAK2^{V617F} HSPC and their reciprocal endogenous WT HSPC from non-irradiated and irradiated BM legs. Microarray analysis showed high mRNA levels of carbonic anhydrase 1 (mouse CAR1) in JAK2^{V617F} HSPC from both legs with the highest mRNA levels in the non-irradiated leg **(****Fig. 4b** and **Fig. 5a****)** associated with a high CAR1 protein levels **(****Fig 4c****).** Of note, in PV patients, high carbonic anhydrase 1 (human CA) mRNA levels were observed by RNA-Seq analysis of JAK2^{V617F} CD34⁺ progenitor cells **(****Fig. 4d****).**

In JAK2^{V617F} CD34⁺ progenitor cells from PV patients, analysis of mRNA levels of all carbonic anhydrases (human CA) showed that only *CA1* and CA2 mRNA levels were increased with *CA1* mRNA levels being by far the highest. **(****Fig. 4n****).**

To assess the contribution of CAR1 to PV development, JAK2^{V617F} LSK cells were transduced with *Carl* shRNA or control shRNA lentiviruses and transplanted into one leg-irradiated recipient mice **(****Fig. 5b****).** Mice transplanted with control shRNA JAK2^{V617F} LSK cells displayed blood parameters associated with PV progression, but mice transplanted with *Carl* shRNA JAK2^{V617F} LSK cells showed no sign of the disease **(****Fig. 4e** and **4f****),** except when non-transduced JAK2^{V617F} LSK cells were the most numerous.

**These results demonstrate that CA1 gene expression is altered in JAK2^{V617F} stem/progenitor hematopoietic cells; and that its increase in CD34⁺ hematopoietic progenitors of PV patients is indicative of a poor prognosis. It also demonstrates that CA1 expression is differentially regulated when compared to other carbonic anhydrase isoforms.**

Daily injections of Furosemide, a specific CAR1 inhibitor that is used as diuretic, as described in Supuran ("How many carbonic anhydrase inhibition mechanisms exist?" J. Enzyme Inhib. Med. Chem. 31, 345-360 (2016)), then resulted in decreased JAK2^{V617F} cell burden in blood **(****Fig. 4g****),** spleen weight **(****Fig. 4h****)** and PV blood parameters **(****Fig. 5b****)** and improved survival rate **(****Fig. 4i****)** in mice transplanted with JAK2^{V617F}BM cells.

The improved survival rate is also reproduced with a second mutant cell-type (ICN1) in the presence of Furosemide **(****Fig. 7****).** ICN1 (Intracellular Notch 1) has been associated to a majority of T-cell acute lymphoblastic leukemia (T-ALL) in the litterature, as shown in Gachet et al. ("Leukemia-initiating cell activity requires calcineurin in T-cell acute lymphoblastic leukemia"; Leukemia (2013) 27, 2289-2300).

Daily injection of Furosemide after PV establishment resulted in a rapid, stable and Furosemide-dependent decrease of blood JAK2^{V617F} cell burden **(****Fig. 4j****)** associated with decreased hematocrit **(****Fig. 4k****)** and spleen weight **(****Fig. 4l****),** survival of the treated mice and a significant decrease of JAK2^{V617F} LT-HSC contribution to the LT-HSC compartment **(****Fig. 4m****).** Because Furosemide is also a diuretic, we treated JAK2^{V617F} mice with Spironolactone, another diuretic with no known activity on CAR1 and found that it had no effect on the survival of mice with PV and PV associated symptoms **(****Fig. 5c** and **5d****).**

**These results demonstrate a previously unreported therapeutic effect of Furosemide, on an animal model of Polycythemia Vera (PV) and other hematopoietic malignancies. These results further indicate that CAR1 is a therapeutic target for suppression of Polycythemia Vera (PV) syndrome in this pre-clinical model through specific targeting of JAK2^{V617F} cells.**

**The therapeutic effect of Furosemide can also be extrapolated to other mutant cell types, including ICN1 mutants.**

**Hence, they serve as evidence for the treatment and prevention of PV and other hematopoietic malignancies, by CAR1 inhibitors such as Furosemide.**

### Example 5.

### Inhibition of carbonic anhydrase 1 by Acetozolamide also suppresses PVprogression.

The experiment is reproduced with a second carbonic anhydrase 1 (CARI) inhibitor (Acetozolamide). Daily injections of Acetozolamide, a second specific CAR1 inhibitor, with the same protocol as Furosemide, resulted in decreased JAK2^{V617F} cell burden in blood **(****Fig. 6a** **- left panel)** and in decreased spleen weight **(****Fig. 6a** **- right panel)** in mice transplanted with JAK2^{V617F} BM cells. A significant decrease of JAK2^{V617F} HSC cell burden in the irradiated and non-irradiated legs is further observed, which is dependent upon the administration of Acetozolamide **(****Fig. 6b****).**

**These results demonstrate that the therapeutic effect of Acetozolamide on the same animal model of Polycythemia Vera (PV) and other hematopoietic malignancies and can be extrapolated to other carbonic anhydrase 1 inhibitors.**

## Claims

1. Carbonic Anhydrase 1 (CA1) inhibitor; for use in a method for treating or preventing a myeloproliferative disorder, an acute myeloid leukemia (AML) and/or a primary or secondary myelofibrosis.

2. Carbonic anhydrase 1 (CA1) inhibitor; for use in a method according to claim 1; wherein the CA1 inhibitor is a sulphonamide or a pharmaceutically acceptable salt thereof.

3. Carbonic Anhydrase 1 (CA1) inhibitor; for use in a method according to claim 1 or 2; wherein the CA1 inhibitor is selected from Furosemide, Acetazolamide, and pharmaceutically acceptable salts thereof.

4. Carbonic Anhydrase 1 (CA1) inhibitor; for use in a method according to any of claim 1 to 3; wherein the CA1 inhibitor is selected from Furosemide and pharmaceutically acceptable salts thereof.

5. Carbonic Anhydrase 1 (CA1) inhibitor; for use in a method according to any of claim 1 to 3; wherein the CA1 inhibitor is selected from Acetazolamide and pharmaceutically acceptable salts thereof.

6. Carbonic Anhydrase 1 (CA1) inhibitor; for use in a method according to claim 1; wherein the CA1 inhibitor is selected from a nucleic acid down-regulating the expression of the gene encoding carbonic anhydrase 1 (*CAR1*)*,* or a transcript thereof.

7. Carbonic Anhydrase 1 (CA1) inhibitor; for use in a method according to any of the preceding claims; wherein the myeloproliferative disorder is selected from: a chronic myeloid leukemia (CML), a chronic neutrophilic leukemia (CNL), polycythemia vera (PV), a primary myelofibrosis (PMF), an essential thrombocythemia (ET), a chronic eosinophilic leukemia.

8. Carbonic Anhydrase 1 (CA1) inhibitor; for use in a method according to any of the preceding claims; wherein it is for treating or preventing a polycythemia vera (PV), an acute myeloid leukemia (AML), a primary myelofibrosis (PMF) and/or a secondary myelofibrosis.

9. Carbonic Anhydrase 1 (CA1) inhibitor; for use in a method according to any of the preceding claims; wherein it is for treating or preventing a primary myelofibrosis (PMF) or a secondary myelofibrosis.

10. Pharmaceutical composition comprising a Carbonic Anhydrase 1 (CA1) inhibitor; for use in a method for treating or preventing a myeloproliferative disorder, an acute myeloid leukemia (AML) and/or a primary or secondary myelofibrosis.

11. *In vitro* or *ex vivo* use of a level of expression or activity of Carbonic Anhydrase 1 (CA1), as a biomarker of a myeloproliferative disorder, an acute myeloid leukemia (AML) and/or a primary or secondary myelofibrosis.

12. *In vitro* or *ex vivo* method for the diagnosis or follow-up of a myeloproliferative disorder, an acute myeloid leukemia (AML) and/or a primary or secondary myelofibrosis in an individual, comprising the steps of:
a) determining a level of expression or activity of Carbonic Anhydrase 1 (CA1) in a biological sample from the individual;
b) comparing the level of expression or activity of CA1 to a reference value, wherein an increase of the level of expression or activity of Carbonic Anhydrase 1 (CA1) is indicative of the occurrence of said myeloproliferative disorder, acute myeloid leukemia (AML) and/or primary or secondary myelofibrosis in the individual.

13. *In vitro* or *ex vivo* method for screening an active agent for the treatment or prevention of a myeloproliferative disorder, an acute myeloid leukemia (AML) and/or a primary or secondary myelofibrosis, comprising the steps of:
a) determining a level of expression or activity of Carbonic Anhydrase 1 (CA1) in a biological sample;
b) comparing the level of expression or activity of CA1 to a reference value, wherein a decrease of the level of expression or activity of Carbonic Anhydrase 1 (CA1) in the biological sample by the active agent is indicative of its efficacy for the treatment or prevention of said myeloproliferative disorder, acute myeloid leukemia (AML) and/or primary or secondary myelofibrosis.

14. The *in vitro* or *ex vivo* method according to claim 13; wherein the active agent is selected from sulphonamides and derivatives thereof; in particular Furosemide, Acetazolamide, and pharmaceutically acceptable salts thereof.

15. The *in vitro* or *ex vivo* method according to claim 12 or 13 or 14; wherein the biological sample is a blood sample that comprises a CD34-positive cell.


SEQUENCE LISTING

<110> COMMISSARIAT A LENERGIE ATOMIQUE ET AUX ENERGIES ALTERNATIVES
      INSERM (INSTITUT NATIONAL DE LA SANTE ET DE LA RECHERCHE MEDICALE)
      UNIVERSITE DE PARIS
      UNIVERSITE PARIS-SACLAY

<120> CARBONIC ANHYDRASE 1 (CA1) INHIBITORS FOR THE TREATMENT OR PREVENTION
OF MYELOPROLIFERATIVE DISORDERS AND OTHER HEMATOPOIETIC MALIGNANCIES, AND AS
BIOMARKER OF MYELOPROLIFERATIVE DISORDERS AND OTHER HEMATOPOIETIC
MALIGNANCIES

<130> PR88911

<160> 6

<170> BiSSAP 1.3.6

<210> 1
<211> 81
<212> RNA
<213> Artificial Sequence

<220>
<223> shRNA

<400> 1
gttctgtatg agaccacagg ggtcccccag aacagtcaga gcctcatttc tcgagaaatg     60

aggctctgac tgttcttttt t                                               81

<210> 2
<211> 78
<212> RNA
<213> Artificial Sequence

<220>
<223> shRNA

<400> 2
gacccacctc ccaaccccga gaaaaaagaa cagtcagagc ctcatttctc gagaaatgag     60

gctctgactg ttctgggg                                                   78

<210> 3
<211> 81
<212> RNA
<213> Artificial Sequence

<220>
<223> shRNA

<400> 3
gttctgtatg agaccacagg ggtcccccgc ttccttcatc tctggattac tcgagtaatc     60

cagagatgaa ggaagctttt t                                               81

<210> 4
<211> 78
<212> RNA
<213> Artificial Sequence

<220>
<223> shRNA

<400> 4
gacccacctc ccaaccccga gaaaaagctt ccttcatctc tggattactc gagtaatcca     60

gagatgaagg aagcgggg                                                   78

<210> 5
<211> 81
<212> RNA
<213> Artificial Sequence

<220>
<223> shRNA

<400> 5
gttctgtatg agaccacagg ggtccccccc gtggatggaa ctagatattc tcgagaatat     60

ctagttccat ccacggtttt t                                               81

<210> 6
<211> 78
<212> RNA
<213> Artificial Sequence

<220>
<223> shRNA

<400> 6
gacccacctc ccaaccccga gaaaaaccgt ggatggaact agatattctc gagaatatct     60

agttccatcc acgggggg                                                   78